# EUROPEAN PATENT APPLICATION

(11) **EP 4 140 476 A1**
(43) Date of publication of application: **01.03.2023**
(21) Application number: 21382781.9
(22) Date of filing: 25.08.2021
(51) Int. Cl.: A61K 9/107, A61K 9/06, A61K 47/10, A61K 47/38, A61K 47/42, A61K 31/439, A61P 17/00, A61Q 15/00

(54) **AN OIL-IN-WATER EMULSION GEL COMPRISING TIOTROPIUM BROMIDE**

(71) Applicant: Health Innovation Technology Transfer, S.L., 08015 Barcelona (ES)
(72) Inventor: SOLÀ-MORALES I SERRA, Oriol, 08015 Barcelona (ES); BUXADÉ FORTUNY, Maria, 08015 Barcelona (ES)
(74) Representative: Aera A/S

(57) **Abstract**

The present invention relates to an oil-in-water emulsion gel comprising tiotropium bromide for topical use. It can be used in cosmetics, as an antiperspirant or in the prevention and/or treatment of hyperhidrosis. After application to the skin nanoparticles are formed and the drug substance penetrates the skin where it exerts its effect. The oil-in-water emulsion gel is composed in such a manner that the drug substance permeates the epidermis and enters into the dermis, but only to a minor extent permeating through the dermis and into the systemic circulation.

## Description

### Field of the invention

The present invention relates to an oil-in-water emulsion gel comprising tiotropium bromide for topical use. It can be used in cosmetics, as an antiperspirant or in the prevention and/or treatment of hyperhidrosis. After application to the skin nanoparticles are formed and the drug substance penetrates the skin where it exerts its effect. The oil-in-water emulsion gel is composed in such a manner that the drug substance permeates the epidermis and enters into the dermis, but only to a minor extent the drug substance permeates through the dermis and into the systemic circulation. In this manner systemic adverse effects are avoided or markedly reduced. The oil-in-water emulsion gel has a desired shelf-life and is regarded as safe.

### Background of the invention

Hyperhidrosis is a medical condition that causes excessive sweating. It is classified as either primary or secondary hyperhidrosis. Although primarily a physical burden, hyperhidrosis can deteriorate quality of life from a psychological, emotional, and social perspective. This excess of sweat happens even if the person is not engaging in tasks that require muscular effort, and it does not depend on the exposure to heat.

Hyperhidrosis can either be generalized or localized to specific parts of the body. Hands, feet, armpits, groin, and the facial area are among the most active regions of perspiration due to the high number of sweat glands in these areas. When excessive sweating is localized, it is referred to as primary hyperhidrosis or focal hyperhidrosis. Primary hidrosis is idiopathic, caused by overactivity of the sympathetic nerves. It only afflicts a limited body area, mostly underarms, palms, soles, or head. While most of the body stays dry, one or two areas drip with sweat. Primary or focal hyperhidrosis may be further divided by the area affected, for instance, palmoplantar hyperhidrosis (symptomatic sweating of only the hands or feet) or gustatory hyperhidrosis (sweating of the face or chest a few moments after eating certain foods).

Excessive sweating involving the whole body is termed generalized hyperhidrosis or secondary hyperhidrosis. Secondary hyperhidrosis results from side effects of certain medication or an underlying medical condition, such as a disorder of the thyroid or pituitary glands, diabetes, tumors, gout, menopause, certain drugs, or mercury poisoning. Secondary hyperhidrosis may start at any point in life.

Hyperhidrosis can also be classified by onset, either congenital or acquired. Primary or focal hyperhidrosis usually start during adolescence or even earlier and seems to be inherited as an autosomal dominant genetic trait.

In the US 15.3 million people are affected by hyperhidrosis, which is 4.8% of the population. Only 51% of the affected people discussed their condition with a health care professional. 75% of the surveyed people report that their condition has had negative impact on their social life, well-being, emotional or mental health. A large part find that excessive sweating is embarrassing and leads to anxiety.

Available treatments include topical aluminum chloride hexahydrate, off-label oral anticholinergics, injectable botulinum toxin or surgery. They all vary in their efficacy, side effects, cost, and ease of use. Recently, a tiotropium bromide preparation has been suggested for topical administration to achieve an antiperspirant effect (CN106137955). It has, however, systemic effects in addition to local effects at the site of application.Tiotropium bromide is an anticholinergic agent. Oral anticholinergic medications such as glycopyrrolate, oxybutynin, benztropine and propantheline are known in the treatment of excessive sweating. However, in general, anticholinergic medications work systemically and cannot target one body area, therefore they decrease sweating over the entire body, even at locations where sweating is not a problem. This overall decrease in sweating can put the patient at risk for overheating. Moreover, these medications produce severe systemic side effects (extreme dry mouth, stomach cramps, urinary problems, etc.) and need to be discontinued.

Thus, there is still a need for developing compositions that target limited body areas that are affected with excessive sweating, and which compositions are easy to use, safe and effective.

### Summary of the invention

In its broadest aspect, the present invention relates to a dermatologically acceptable oil-in-water emulsion gel comprising tiotropium bromide. It also relates to the use of such a gel in cosmetics, as an antiperspirant or in the prevention and/or treatment of hyperhidrosis.

The invention also relates to a method for preparing an oil-in-water emulsion gel comprising tiotropium bromide, the method comprising
i) preparing an hydroalcoholic gel comprising one or more water-soluble cellulose derivatives,
ii) preparing a organic phase comprising tiotropium bromide,
iii) mixing the hydroalcoholic gel with the organic phase to obtain said oil-in-water emulsion gel.

### Detailed description of the invention

As mentioned above, the present invention relates to tiotropium bromide for use as an antiperspirant or for use in the treatment or prevention of hyperhidrosis by applying tiotropium bromide topically and delivering it into the skin. Tiotropium is an anticholinergic agent, but in contrast to the general understanding that anticholinergic agents must act systemically, the present inventors have found that tiotropium bromide must act locally within the skin directly on the sweat glands.

The present invention also relates to a composition comprising tiotropium bromide for topical use, the composition being in the form of a dermatologically acceptable oil-in-water emulsion gel comprising tiotropium bromide.

In a first aspect, the invention relates to a dermatologically acceptable oil-in-water emulsion gel comprising tiotropium bromide.

### Definitions

Emulsion gels are a class of soft solid-like materials. These composite materials are structurally either a polymeric gel matrix into which emulsion droplets are incorporated (emulsion-filled gels), or a network of aggregated emulsion droplets (emulsion particulate gels).

In the present context, an oil-in-water emulsion gel is a composition comprising a hydroalcoholic gel into which an organic phase containing tiotropium bromide is incorporated, thus creating oil droplets inside the aqueous gel phase (as an emulsion), and wherein nanoparticles are formed *in situ* after application to the skin. As mentioned above, the nanoparticles are formed on the skin after applicatin of the composition due to evaporation of ethanol (*in situ* self assembled nanoparticles). *In situ* self assembled nanoparticles are described in WO 2013/120856. The nanoparticles are a slow release system that degrades over time to slowly release the tiotropium bromide that gradually penetrates into the skin.

In the present context, a gel is defined as a semi-solid that can have properties ranging from soft and weak to hard and tough. Gels are defined as substantially dilute cross-linked systems, which exhibit no flow when in steady state. By weight, gels are mostly liquids, yet they behave like solids because of a three-dimensional network within the liquid. A gel is typically formed by use of a gelling or swelling agent and a liquid.

In the present context, the term "nanoparticle" may be a matrix nanoparticle, wherein said matrix nanoparticle comprises a matrix comprising a plant protein and at least a water-miscible non-volatile solvent, or it may be a core-shell vesicular nanoparticle, wherein said core-shell vesicular nanoparticle comprises a core and a shell, and the shell comprises a plant protein and at least one miscible non-volatile solvent.

In the present context, the term "organic phase" is used to denote a composition comprising one or more organic substances, such as an oil substance. One example thereof is oleic acid. Upon admixing with an aqueous-containing composition it may form an emulsion.

In the present context, the term "tiotropium bromide" denotes the drug substance tiotropium bromide as well as other pharmaceutically acceptable salts of tiotropium and tiotropium as a free base.

The term "dermatologically-acceptable", as used herein, means that the compositions or components thereof so described are suitable for use in contact with mammalian epidermal tissue without undue toxicity, incompatibility, instability, allergic response, and the like. A dermatologically acceptable formulation should preferably also have a good cosmetic appearance, drying time and spreadability.

### Hydroalcoholic gel - before adding an organic phase comprising tiotropium bromide

The hydroalcoholic gel into which the organic phase is dispersed typically comprises a gelling agent, one or more solvents and optionally one or more pharmaceutically acceptable excipients.

The gelling agent is a water-soluble polymer such as a polymer selected from cellulose derivatives or poly(acrylic acids) or co-polymers with acrylic acid. Suitable cellulose derivates include hydroxyethylcellulose, hydroxypropylcellulose and hydroxypropyl methylcellulose. Suitable polymers containing acrylic acid include poly(acrylic acid) such as Carbopol.

Solvents for use in the hydroalcoholic gel include water, ethanol, propylene glycol and glycerol, coco-caprylate/caprate (e.g.Kollicream^{®} 3C), isopropyl alcohol, triacetin, dimethyl formamide.

A hydroalcoholic gel may also contain one or more pharmaceutically acceptable excipient such e.g. a permeation enhancer. Suitable permeation enhancers include oleic acid, diethylene glycol monoethyl ether (e.g.Transcutol HP), dimethyl isosorbide (DMI), isopropyl myristate (IPM), dimethyl sulfoxide (DMSO), and Kollicream^{®} 3C.

### Organic phase comprising tiotropium bromide - before adding it to the hydroalcoholic gel

An organic phase is formed comprising tiotropium bromide and optionally a water-insoluble polymer, as well as one or more solvents capable of dissolving the polymer (if included).

When the organic phase and the hydroalcoholic gel are mixed, an emulsion may be formed between the aqueous phase (gel) and the oil phase. The oil phase is finely dispersed in the aqueous phase (oil-in-water gel emulsion).

Examples of suitable water-insoluble polymers are plant proteins such as plant polymers belonging to a class of prolamine proteins. Zein is a plant protein isolated from corn or maize belonging to the family of prolamines, which are composed of high amounts of hydrophobic amino acids such as proline, glutamine and asparagine. Zein is clear, non-toxic, biodegradable and water-insoluble vegetable protein. Zein has been investigated and used as a polymer in the pharmaceutical, medical food, cosmetic, adhesive and packaging industries.

Examples of suitable solvents for the water-insoluble polymers are propylene glycol, ethanol, Transcutol HP, polyethylene glycol, 1,3-propanediol, benzyl alcohol, dimethyl sulphoxide and mixtures thereof.

The organic phase may also contain one or more pharmaceutically or topically acceptable excipients such as e.g. one or more surfactants, one or more penetration enhancers and/or one or more polymer plasticizers.

In order to establish an emulsion or to ensure homogeneous distribution of the oily phase in the oil-in-water emulsion gel, either the hydroalcoholic gel or the organic phase (or both) may contain one or more surfactants. Suitable surfactants for use are non-ionic surfactants such as polysorbates (Tween 20, Tween 40, Tween 60, Tween 80), polyoxyglycerides (Labrasol^{®}), and polyethoxylated castor oil (such as Cremophor EL, Cremophor RH40).

Suitable permeation enhancers that may be included in the hydroalcoholic gel or in the organic phase or in both are oleic acid, Transcutol, dimethyl isosorbide (DMI), isopropyl myristate (IPM), DMSO, Kollicream^{®} 3C and mixtures thereof.

Suitable plasticizers include oleic acid and caprylocapryl polyoxyl-8-glycerides (e.g. Labrasol).

### Oil-in-water emulsion gel

The composition to be topically applied is an oil-in-water emulsion gel. The nanoparticles comprising tiotropium bromide are formed *in situ* on the skin after evaporation of a solvent. The mean particle size of the particles is at the most 500 nm such as at the most 450 nm, at the most 400 nm or at the most 350 nm, the particle size being measured using a Light Scattering equipment and wherein 100 µL of the gel is diluted to 5 mL with water.

The polydispersity index of the oil-in-water emulsion gel is at the most 0.5 such as at the most 0.45, at the most 0.4 or at the most 0.35, the polydispersity index being determined using a Light Scattering equipment and wherein 100 µL of the gel is diluted to 5 mL with water.

An oil-in-water emulsion gel of the invention may comprise a plant protein as mentioned above under the heading "Organic phase comprising tiotropium bromide". The plant protein is a polymer that is not soluble in water. The nanoparticles formed when including the plant protein contain the plant protein, and tiotropium bromide is either trapped in the nanoparticles or it is adhered to the surface of the nanoparticles. In the case, where the nanoparticles are formed *in situ,* tiotropium bromide is adhered to the plant protein in the oil-in-water emulsion gel and after formation of the nanoparticles, tiotropium bromide is either encapsulated in the nanoparticles or adhered to the surface of the nanoparticles.

To form nanoparticles the plant protein (if included) is preferably present together with a water-miscible or water-soluble solvent. Suitable solvents are described above and are propylene glycol, ethanol, Transcutol HP, polyethylene glycol, 1,3-propane diol, benzyl alcohol, dimethyl sulphoxide and mixtures thereof.

In one embodiment, the concentration of said water-insoluble polymer (plant protein) in the oil-in-water emulsion gel is from about 0.05% to about 10% w/w such as from about 0.1% to about 8% w/w, from about 0.2% to about 5% w/w, from about 0.2% to about 2% w/w, from about 0.2% to about 1.5% w/w or from about 0.5% to about 1% w/w, based on the total weight of the gel.

An oil-in-water emulsion gel of the invention comprises tiotropium bromide. In one embodiment, the concentration of tiotropium bromide in the oil-in-water emulsion gel is from about 0.01% to about 5.0% w/w, such as from about 0.025% to about 3.5% w/w, from about 0.05% to about 2.5% w/w, from about 0.1% to about 2.5% w/w, from about 0.5% to about 2.5% w/w such as from about 0.75% to about 2.0% w/w, based on the total weight of the oil-in-water emulsion gel.

### Tiotropium bromide

Tiotropium bromide is a long-acting, antimuscarinic bronchodilator used in the management of chronic obstructive pulmonary disease (COPD) and asthma. It is freely soluble in dimethyl sulphoxide, soluble in methanol, sparingly soluble in water and practically insoluble in methylene chloride. The solubility in aqueous solutions at room temperature is approx. 2.5%, independent of pH. Its chemical name is [(1*S*,2*S*,4*R*,5*R*)-9,9-dimethyl-3-oxa-9-azoniatricyclo[3.3.1.0^{2,4}]nonan-7-yl] 2-hydroxy-2,2-dithiophen-2-ylacetate bromide. Its structural formula is

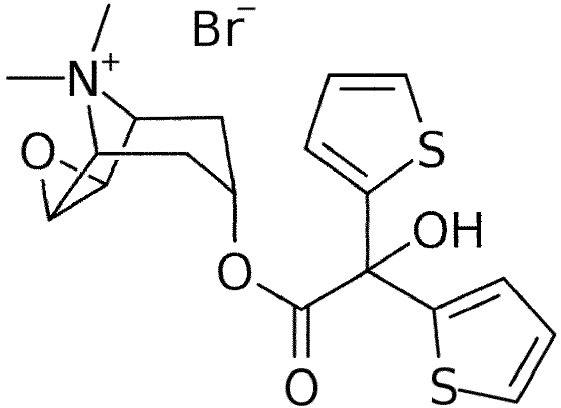

It is a white to yellowish-white, odorless crystalline powder. It is sold under the name Spiriva^{®} and other names. It is a muscarinic receptor antagonist, often referred to as an antimuscarinic or anticholinergic agent.

*Oil-in-water emulsion gel, cont.*

To form a gel, one or more gelling agents are present in an oil-in-water emulsion gel of the invention. Typically, the gelling agent is water-soluble or swells in contact with water.

A water-soluble polymer is selected from cellulose derivatives or acrylic acid polymers or co-polymers.

A suitable cellulose derivative may be selected from hydroxyethyl cellulose, hydroxypropyl cellulose and hydroxypropyl methylcellulose, an an acrylic acid polymer may be a poly(acrylic acid).

The concentration of said water-soluble polymer in the oil-in-water emulsion gel is from about 0.5% to about 5% w/w such as from about 0.5% to about 2.5% or from about 0.75% to about 1.5% w/w based on the total weight of the oil-in-water emulsion gel.

An oil-in-water emulsion gel according to the invention may comprise one or more permeation enhancer, one or more plasticizers, one or more surfactants, one or more gelling agents, one or more solvents besides the water-miscible solvents mentioned above. Examples of such excipients suitable for use in an oil-in-water emulsion gel of the present invention are given herein before. Ethanol is typically contained in an oil-in-water emulsion gel of the invention. Ethanol serves the purpose of being a suitable solvent for the water-insoluble polymer (plant protein) as well as enhancing permeation to some degree. When ethanol evaporates from the oil-in-water emulsion gel e.g. after application, nanoparticles will be formed based on the water-insoluble polymer.

A surfactant may be present in the oil-in-water emulsion gel. The function of a surfactant is typically to lower the surface tension between two liquids or between a solid and a liquid. As a surfactant both contains hydrophobic groups and hydrophilic groups it contains both a water-insoluble (or oil-soluble) component and a water-soluble component and makes an interface between two components that are not miscible. Preferably, a surfactant suitable for use in an oil-in-water emulsion gel according to the invention is a non-ionic surfactant such as those mentioned herein before. The concentration of a surfactant in the oil-in-water emulsion gel is at the most 15% w/w such as at the most 10% w/w, at the most 7.5% w/w, at the most 7% w/w, based on the total weight of the oil-in-water emulsion gel.

In those cases, where the surfactant is a polysorbate (such as Tween^{®}), the concentration of the polysorbate in the oil-in-water emulsion gel is at the most 10% w/w such as at the most 7.5% w/w, at the most 7% w/w, based on the total weight of the oil-in-water emulsion gel.

In those cases, where the surfactant is a polyoxyglyceride (such as Labrasol^{®}), the concentration or said polyoxyglyceride is at the most 3% w/w.

An oil-in-water emulsion gel of the invention may contain one or more surfactants.

As mentioned above, an oil-in-water emulsion gel of the invention may also comprise a permeation enhancer. Examples of suitable permeation enhancers for use in the present invention are given above and includes oleic acid, Transcutol^{®} HP, dimethyl isosorbide (DMI), isopropyl myristate (IPM), DMSO and Kollicrean^{®} 3C. In general, the concentration of said permeation enhancer in an oil-in-water emulsion gel of the invention is at the most 20% w/w, such as at the most 5% w/w based on the total weight of the oil-in-water emulsion gel. In one embodiment, the concentration of permeation enhancer is in the range 0.1 to 20% (w/w). In another embodiment, the concentration of permeation enhancer is in the range 0.2 to 10% (w/w). In still another embodiment, the concentration of permeation enhancer is in the range 0.3 to 5.0% (w/w). In yet another embodiment, the concentration of permeation enhancer is in the range 0.5 to 3.0% (w/w). In those cases, where ethanol is included, ethanol has more than one function as it acts both as a permeation enhancer to some degree and as a solvent. Ethanol is typically present in an oil-in-water emulsion gel of the invention in a concentration of from 25% to about 55% w/w based on the total weight of the oil-in-water emulsion gel. The amounts of permeation enhancer provided above do not include the amount of ethanol. If ethanol is replaced with another volatile solvent and ethanol is only included as a permeation enhancer, the concentration of ethanol is at the most 10% w/w based on the total weight of the oil-in-water emulsion gel.

An oil-in-water emulsion gel of the invention comprises a solvent. Typical solvents include propylene glycol, glycerol, ethanol, and water. In one embodiment, the concentration of water is from 5% to 15% w/w based on the total weight of the oil-in-water emulsion gel. In another embodiment, ethanol is present in a concentration of from 25% to 55% w/w, such as from 30% to 45% w/w based on the total weight of the oil-in-water emulsion gel. In a further embodiment, glycerol is present in a concentration of from 7.5% to 15% w/w based on the total weight of the oil-in-water emulsion gel. In still another embodiment, propylene glycol is present in a concentration of from 15% to 30% w/w based on the total weight of the oil-in-water emulsion gel. In yet another embodiment, the concentration of water is from 5% to 15% w/w, ethanol in a concentration of from 25% to 55% w/w, such as from 30% to 45% w/w, glycerol in a concentration of from 7.5% to 15% w/w, and propylene glycol in a concentration of from 15% to 30% w/w based on the total weight of the oil-in-water emulsion gel.

An oil-in-water emulsion gel according to the invention may also contain one or more further topically acceptable excipients, such as, e.g., preservatives, emollients, pH-adjusting agents, flavors, viscosity-adjusting agents etc.

Specific embodiments of the invention include
i) An oil-in-water emulsion gel comprising
   from 0.01% to 5.0% w/w of tiotropium bromide,
   from 0.05% to 10% of a plant polymer,
   from 1% to 15% w/w of a non-ionic surfactant,
   from 0.5% to 3% w/w of a permeation enhancer (if ethanol is present it is included in the concentration of solvents)
   from 0.5% to 5% of a water-soluble polymer, and
   from 50% to 80% w/w of one or more solvents;
ii) An oil-in-water emulsion gel comprising
   from 0.05% to 3.5% w/w tiotropium bromide,
   from 0.25% to 2.0% w/w of a water-insoluble polymer such as zein,
   from 20% to 30% w/w of a solvent for water-soluble polymers such as propylene glycol,
   from 5% to 10% w/w of a non-ionic surfactant such as polysorbate 20,
   from 2% to 3% w/w of a non-ionic surfactant such as Labrasol^{®} (caprylocapryl polyoxyl-8-glycerides),
   from 1.75% to 2.5% w/w of a fatty acid such as oleic acid,
   from 2% to 3% w/w of a permeation enhancer such as Transcutol^{®} (diethylene glycol monoethyl ether) and/or dimethyl isosorbide,
   from 0.75% to 1.25% w/w of a water-soluble polymer such as hydroxypropylcellulose,
   from 10% to 15% w/w of a solvent such as glycerol,
   from 30% to 40% w/w of a solvent such as ethanol, and
   from 7.5% to 13% w/w of a solvent such as water;
iii) An oil-in-water emulsion gel comprising
   from 1.0% to 2.5% tiotropium bromide,
   from 0.25% to 1.0% w/w of zein,
   from 20% to 30% w/w of propylene glycol,
   from 5% to 10% w/w of polysorbate 20,
   from 1.75% to 2.5% w/w oleic acid,
   from 2% to 3% w/w of caprylocapryl polyoxyl-8-glycerides,diethylene glycol monoethyl ether and/or dimethyl isosorbide,
   from 0.75% to 1.25% w/w of hydroxypropylcellulose,
   from 30% to 40% w/w of ethanol,
   from 10% to 15% w/w of glycerol, and
   from 7.5% to 13% w/w of water.

### Use of an oil-in-water emulsion gel of the invention

An oil-in-water emulsion gel according to the invention can be used in the treatment or prevention of hyperhidrosis; it can be used as an anti-perspirant and it can be used in cosmetics. A particular feature of an oil-in-water emulsion gel of the invention is that the gel is administered topically to deliver tiotropium bromide to the dermis of the skin, where it exerts its therapeutic effect locally on sweat glands. As seen from the examples herein an oil-in-water emulsion gel only to a minor extent permeates the skin and enters into the systemic circulation.

### Method for preparing an oil-in-water emulsion gel of the invention

The present invention also relates to a method for preparing an oil-in-water emulsion gel comprising tiotropium bromide, the method comprising
i) preparing an hydroalcoholic gel comprising one or more water-soluble polymers,
ii) preparing an organic phase comprising tiotropium bromide,
iii) mixing the hydroalcoholic gel with the organic phase to obtain said oil-in-water emulsion gel.

All details mentioned herein before regarding the hydroalcoholic gel, organic phase, and oil-in-water emulsion gel apply *mutatis mutandis* for this aspect of the invention.

### Preparation of a hydroalcoholic gel

The hydroalcoholic gel is prepared by dissolving the water-soluble polymer in water, then adding ethanol and optionally other topically acceptable agents.The water-soluble polymer is typically a cellulose derivative or an acrylic acid polymer as described herein before.

As mentioned above, one or more topically acceptable agents may be added when preparing the hydroalcoholic gel such as propylene glycol, glycerol, hydroxypropyl cellulose, a permeation enhancer.

### Preparation of an organic phase

An organic phase is prepared by dissolving tiotropium bromide in propylene glycol and adding a water-insoluble polymer, and further adding one or more topically acceptable excipients such as e.g. a non-ionic surfactant, a permeation enhancer, and optionally other topically acceptable agents.

### General

It should be understood that any feature and/or aspect discussed above in connections with the oil-in-water emulsion gel apply by analogy to the other aspects of the invention described herein. The following figures and examples are provided below to illustrate the present invention. They are intended to be illustrative and are not to be construed as limiting in any way.

### Brief description of the figures

Figure 1 shows the 4 steps in the preparation of an oil-in-water emulsion gel of the invention. Step 1 is the design of an hydroalcoholic gel, step 2 is design of an organic phase containing tiotropium bromide, step 3 is incorporation of the organic phase into the hydroalcoholic gel to form the oil-in-water emulsion gel and step 4 is application of the oil-in-water emulsion gel to the skin.
Figure 2 shows a Franz diffusion cell
Figure 3 shows the distribution of tiotropium bromide (TTB) in skin (A) and RC (B) six hours after product administration on porcine skin. Statistically differences were assessed by Kruskal Wallis. *: p<0.05; **p <0.01; ***: p<0.001; IC: internal control

### Methods, Materials and Examples

### Equipment

HPLC Agilent 1260 Infinity, binary pump G1312B, autosampler G1367E, thermostat G1330B, DAD detector G4212B equipped with a high sensitivity cell, thermostatized column compartment
G1316A. Controlled by Agilent OpenLab CDS software, version A.02.08 SP1
Automatic Franz Diffusion Cells. Hanson Corporate.
Particle size analyzer. Brookhaven, 90 Plus
Optical Microscope. Nikon, Eclipse Ci-L
Climatic Chambers. Memmert, HPP 108
Analytical balance. Mettler Toledo, XA 204 Delta Range
Water purification system. Millipore, Direct Q 3UV
Dermatome. Braun, Acculan 3Ti
Precision thickness gauge. Baxlo precision, model 3000
Autoclave. Raypa, AH-21N2.
Laboratory stove. Indelab, IDL-CD-120.
Digital ultrasonic bath. Bandelin, Sonorex Digitec DT100H
Extraction hood. Indelab, Flowlan GN
pH-meter. Progen Scientific, PL-700PV
-20°C Freezer. Liebherr, GX823
Refrigerator. Beko, CN 232102
Micropipettes
Others (beakers, test tubes, eppendorf tubes, HPLC vials, volumetric flasks, graduated cylinders, syringes, Falcon tubes, etc.)

### Reagents

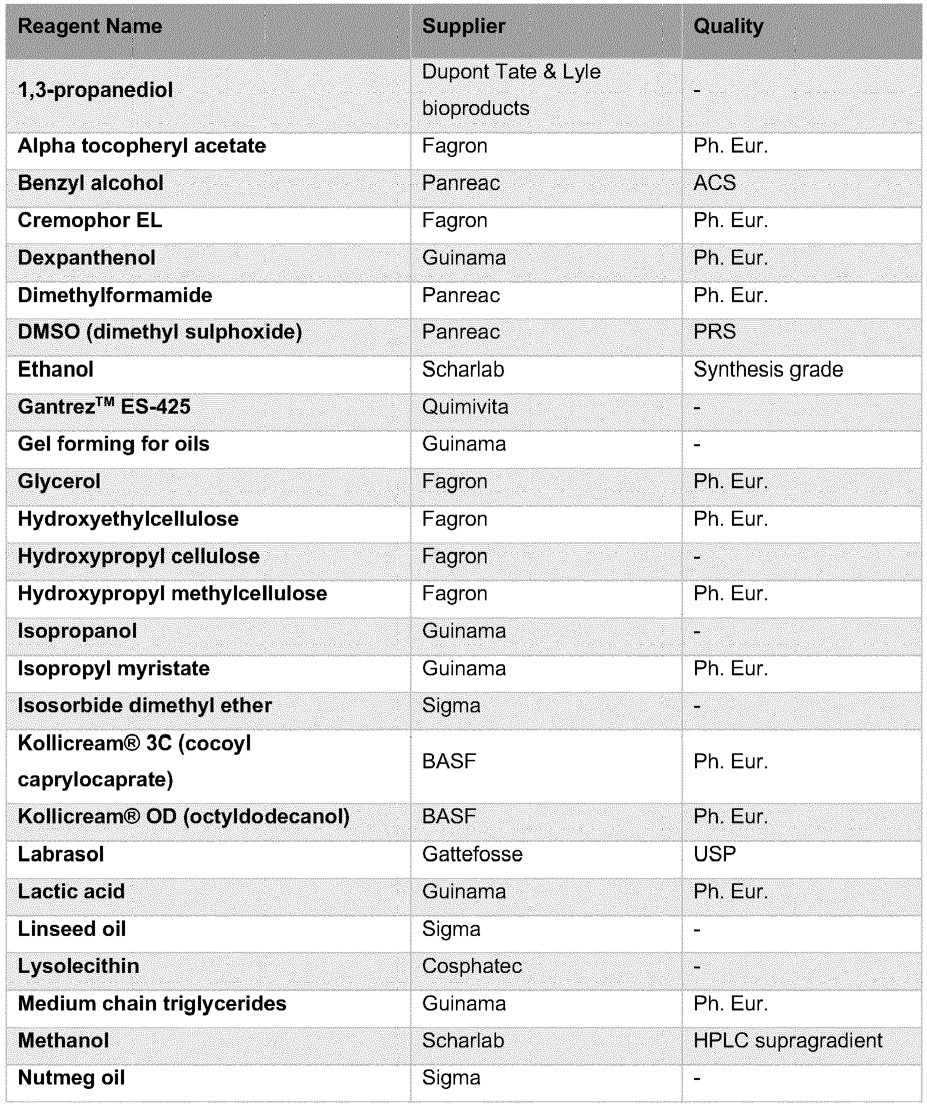

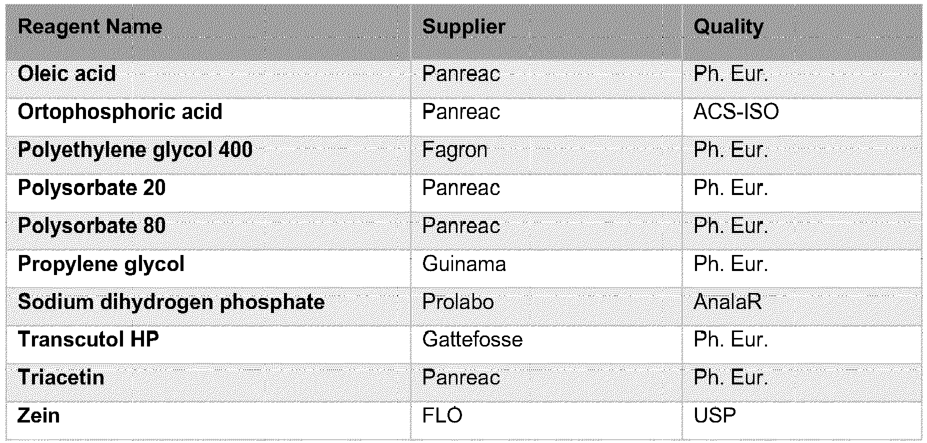

### Procedure

### 1. Analytical method implementation

A novel analytical method was developed and implemented in order to proceed to the identification and subsequent quantification of tiotropium in the developed formulations.

For that purpose an Agilent 1260 Infinity HPLC was used, equipped with a quaternary pump, thermostatized column compartment and a high sensitivity flow cell (60 mm optical path length).

The chromatographic conditions were as follows:
- Analytical Column: Agilent Zorbax SB-Aq (150x4.6mm) 5µm
- Column Temperature: 40 °C
- Injection Volume: 20 µL
- Flow Rate: 1.0 mL/min
- Detection UV Wavelength: 237 nm
- Aqueous Mobile Phase (A): Phosphate Buffer pH= 3.00
- Organic Mobile Phase (B): Methanol
- Mobile Phase Mixture: 70 % A + 30 % B (isocratic mode)
- Run time: 15 minutes

The active was extracted from the formulations by vigorous stirring of an accurately weighed amount of product in a mixture phosphate buffer: methanol (70:30). Once the formulation and subsequently tiotropium were solubilized, the mixture was transferred to a volumetric flask and filled to the mark with the same solution.

### 2. Solubility, compatibility and excipient selection

In order to select the most suitable excipients for the encapsulation of the active, different solvents and surfactants were used: ethanol, isopropanol, propylene glycol, benzyl alcohol, 1,3-propanediol, polyethylene glycol (e.g.PEG 400), triacetin, cocoyl caprylocaprate (Kollicream^{®} 3C), octyldedecanol (Kollicream^{®} OD), diethylene glycol monoethyl ether (Transcutol HP), oleic acid, isopropyl myristate, medium chain triglycerides (MCT), dimethyl formamide, polysorbate 20, polysorbate 80, labrasol, macrogolglycerol ricinoleate (cremophor EL), linseed oil and dimethyl sulphoxide (DMSO) among others.

Regarding the encapsulating polymers to be used, both maleic anhydride polymers and copolymers such as polymers of monoalkyl esters of poly(methylvinylether/maleic anhydride) (e.g.butyl ester of PVM/MA copolymer, Gantrez^{™} ES-425) and zein were evaluated as candidates. Finally, zein was selected as the most adequate one.

For the evaluation of the compatibility of the active in each excipient, 25 mg of tiotropium bromide (TTB) were weighed in suitable glass containers and increasing amounts of the appropriate solvent were added. The samples were subjected to heating and ultrasonication when necessary. The appearance of the mixture and the maximum concentration of TTB in each solvent was recorded.

### 3. Formulation design and development

One of the main goals of this project was to develop a product with elegant cosmetic properties. Therefore, one of the first steps was to design a cosmetically acceptable hydroalcoholic gel into which the organic phase containing the active would be incorporated. For this purpose, several gelling agents were evaluated and the physicochemical characteristics of the obtained gels were studied.

In parallel, different surfactants, co-surfactants and solvents were added to different concentrations of zein or Gantrez^{™} ES-425 dissolved in propylene glycol or Transcutol HP in order to evaluate the miscibility between the solvent mixtures. The most suitable ratio surfactant/s-co-surfactant/s was selected. Organic phases containing Gantrez^{™} ES-425 had to be discarded as TTB precipitates when in contact with this encapsulating polymer. However, all the organic phases containing zein were clear and did not show any phase separation, crystallization or aggregation.

Mixtures with turbid appearance were discarded and only transparent mixtures were chosen to dissolve or disperse tiotropium bromide. Subsequently, the formulations were added to the suitable gel candidates and the encapsulation systems were characterized.

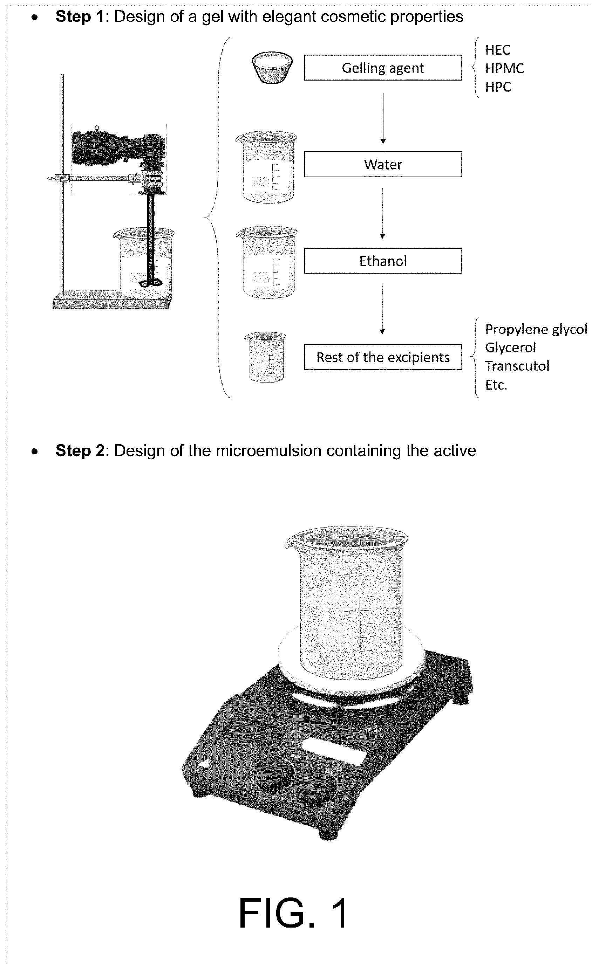

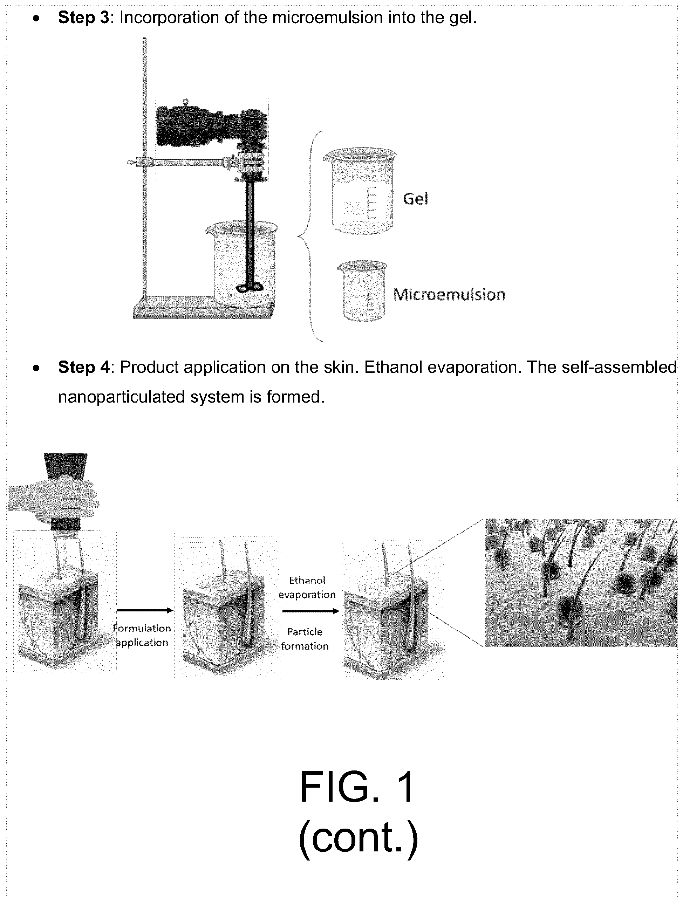

The procedure is outlined in Figure 1.

### 4. Optimization and physicochemical characterization

As previously described, the formulations obtained were characterized and evaluated according to the methodology described below:

### 4.1 Macroscopical appearance

Both the organic phase and the gelled products were observed macroscopically. In both cases the formulations that presented aggregates, phase separation and/or turbidity were discarded.

### 4.2 Particle size measurement

The size of the particles was measured using a particle size analyzer (90S, Brookhaven). For that purpose, 100 µL of each formulation (gelled product) were diluted to 5 mL with water. The products that formed aggregates upon contact with water were discarded as well as those that formed particles greater than 350 nm or showed a polydispersity over 0.35.

### 4.3 Stability

Different stability tests were carried out to select the most suitable formulations:
- *Screening test:* The preliminary stability of the formulations was evaluated after 5 days of storage at 4°C (active quantification by HPLC-UV, absence of aggregates, adequate macroscopical appearance, and no phase separation).
- *Stability under stress conditions:* The final formulations (or gelled products) were subjected to four stress cycles, each of them consisting of two hours heating at 75 °C in the autoclave followed by overnight storage at -20 °C. After the last cycle was completed, the stability was assessed by evaluation of the macroscopical appearance as well as active quantification by HPLC-UV.
- *Long term* & *accelerated stability:* The final formulations (or gelled products) were stored under the following conditions: 4 °C, 25 °C/60% RH and 40°C/75% RH. Their stability was assessed by evaluation of the macroscopical appearance as well as active quantification by HPLC-UV on regular intervals.

### 4.4 Ex-vivo percutaneous absorption study

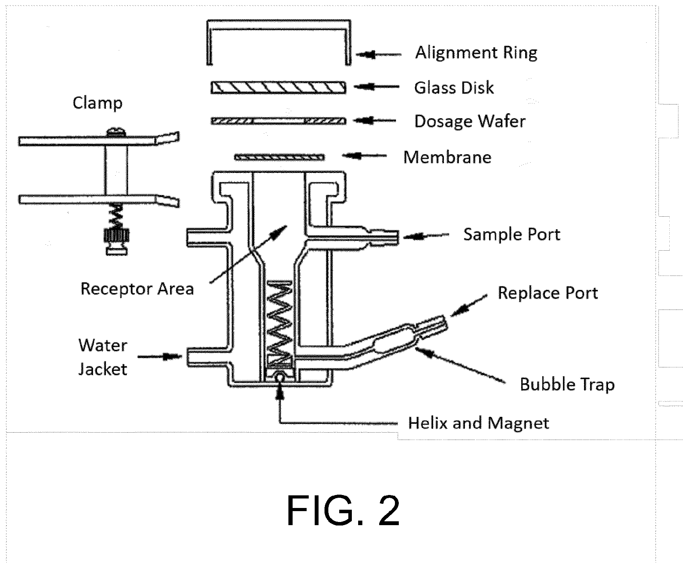

This type of studies in Franz diffusion cells allow for the evaluation of the degree of permeation of a certain chemical entity through the skin into the receptor compartment. A piece of porcine skin was placed on the cell (see Figure 2) and in contact with the fluid of the receptor chamber. The product to be evaluated was applied on the surface of the skin and the system was maintained under stirring at 32 ± 1 °C throughout the desired time period.

At the end of the study, the receptor chamber fluid was collected, the skin removed and washed with the adequate medium to remove any non-absorbed excess product. All the specimens were analyzed in order to determine their TTB content.

Several preliminary *ex vivo* percutaneous absorption studies were performed in order to compare the permeation behaviour of the different prototypes.

Firstly, a 22 hour-long Franz diffusion cell study was carried out (with sample collection at 0, 0.5, 1, 2, 4, 6, 8, 12 and 22h) in order to establish the time-point at which differences in permeation could be observed between a formulation with the highest permeation capacity (TTB directly dissolved in DMSO) and a formulation with an expected low permeation profile.

The high number of prototypes to be tested made it impossible to perform the standard Franz Diffusion cell studies and therefore a high-throughput alternative was applied, the mini-cells. This system consisted of glass vials with a known volume of PBS and a small magnetic stirrer onto which a piece of porcine skin was placed. The system was sealed with a rubber band, the formulation was applied (10 µL/cm²) and then placed on a magnetic stirrer inside a laboratory stove set at 32 °C until the end of the study. Six replicates of each prototype were performed and the concentration of active in receptor fluid, skin and wash fluid was determined by HPLC-UV.

### 4.5 Statistical analysis

The differences between the prototypes were evaluated using a Kruskal-Wallis test with Dunn's multiple comparison *post-test* and considered significant when p < 0.05. The statistical analysis was performed using GraphPad Software.

### Results

The solubility of the active was determined in different excipients (polymer solvents, permeation enhancers, surfactants, emollients, etc.). Table 1 summarizes the maximum solubility achieved in each of them.

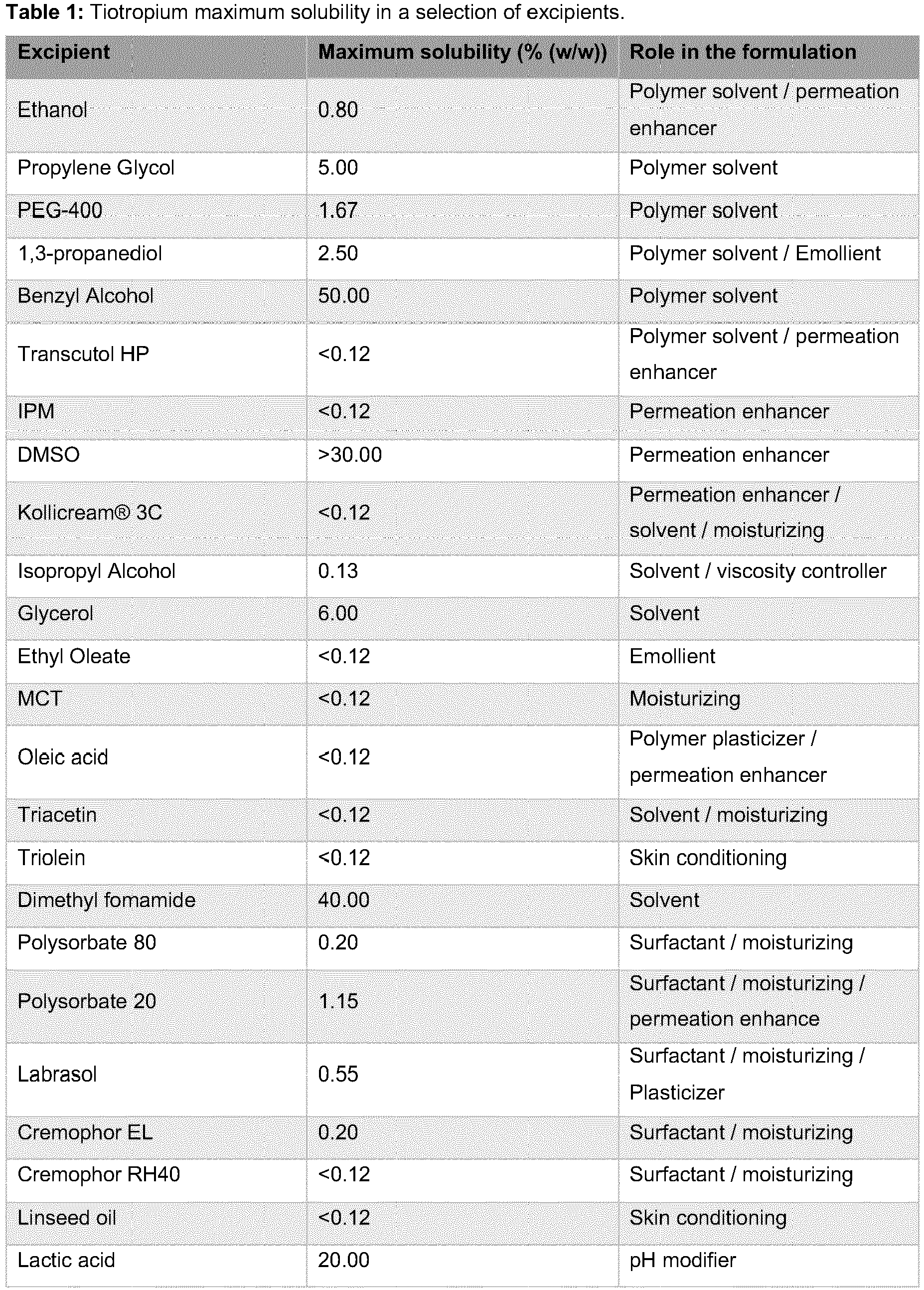

With the information provided by the previous solubility study, the next focus was the development of a gel with elegant cosmetic properties containing TTB-compatible excipients. Thus, two main strategies were followed:

### Strategy 1: Oil-in-water emulsion gel

A gel was designed in which the nanoparticles were formed on the skin after product application due to the evaporation of ethanol (*in situ* self assembled nanoparticles). A significant amount of ethanol was considered beneficial due to the subsequent increase of TTB concentration after its evaporation.

On a first approach, carbopol was evaluated as gelling agent but it was found to be incompatible with the active.

An exhaustive search for gelling agents compatible with a high concentration of ethanol was performed and finally three main cellulose derived gelling agents were selected. Using a fixed concentration of gelling agent (hydroxyethyl cellulose, hydroxypropyl cellulose or hydroxypropyl methylcellulose), several ethanol:water and PG:glycerol ratios were assayed in order to select the combination with best cosmetic properties. These combinations can be found on table 2 below.

The initial testing was performed without TTB in order to evaluate the cosmetic properties of the formulations on skin.

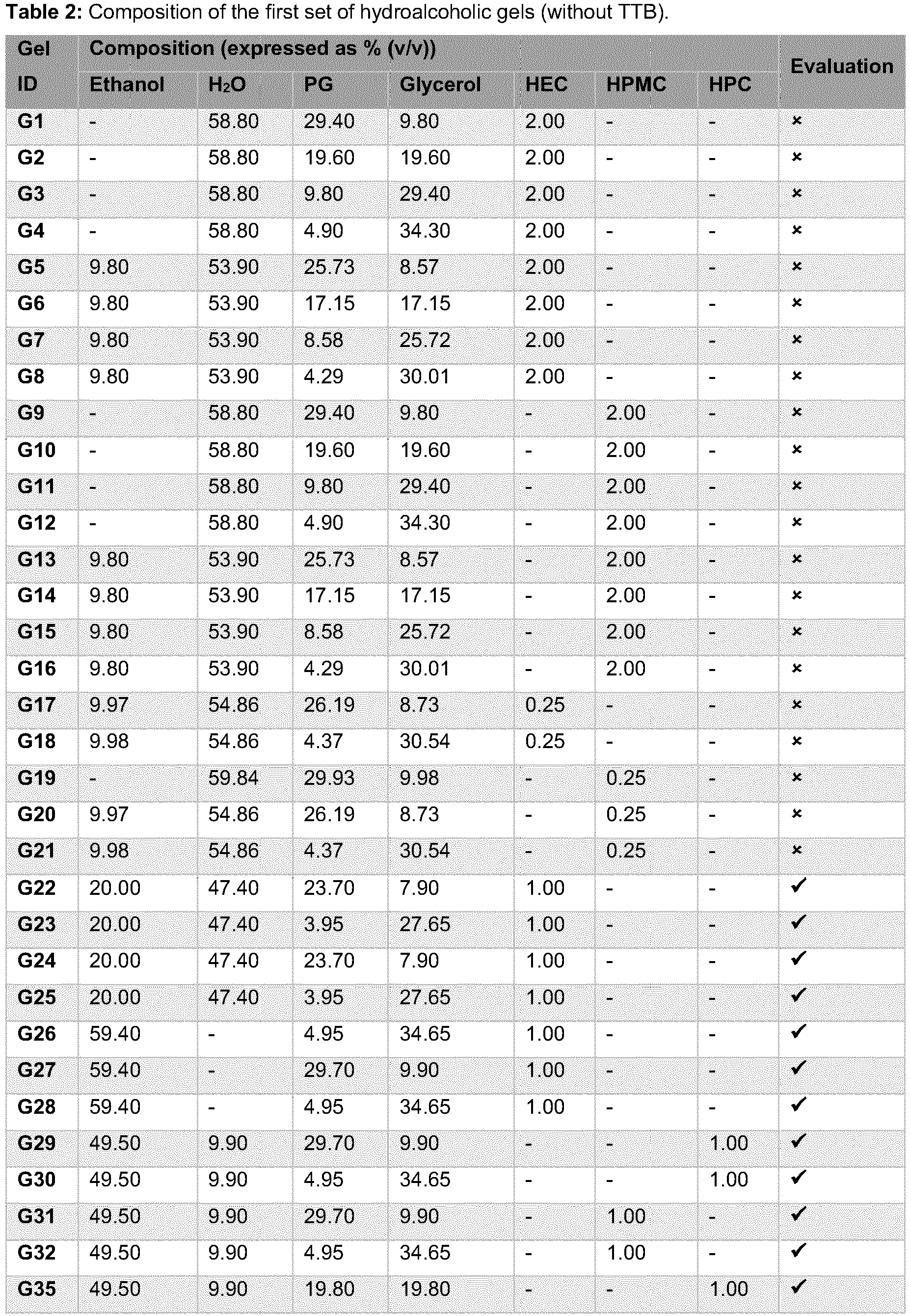

All the preparations were subjected to cosmetic evaluation and it was concluded that the optimal concentration of gelling agent was about 1% such as in a range of from about 0.5% to about 5%. Higher amounts yielded too consistent products that were difficult to spread and needed a long time to dry. On the other hand, lower amounts were not sufficient to provide the gel-like product that is required.

In addition, low ethanol concentrations produced sticky gels and therefore its concentration had to be increased. A major incompatibility between HEC and ethanol (in concentration greater than 25%) was observed.

Gels G29 and G35 were selected as the best ones in terms of cosmetic appearance, drying time and spreadability.

Isopropyl myristate (IPM), Transcutol HP and Kollicream^{®} 3C were then chosen as permeation enhancers to evaluate their compatibility with the previously selected gels. The obtained combinations can be found in table 3 below.

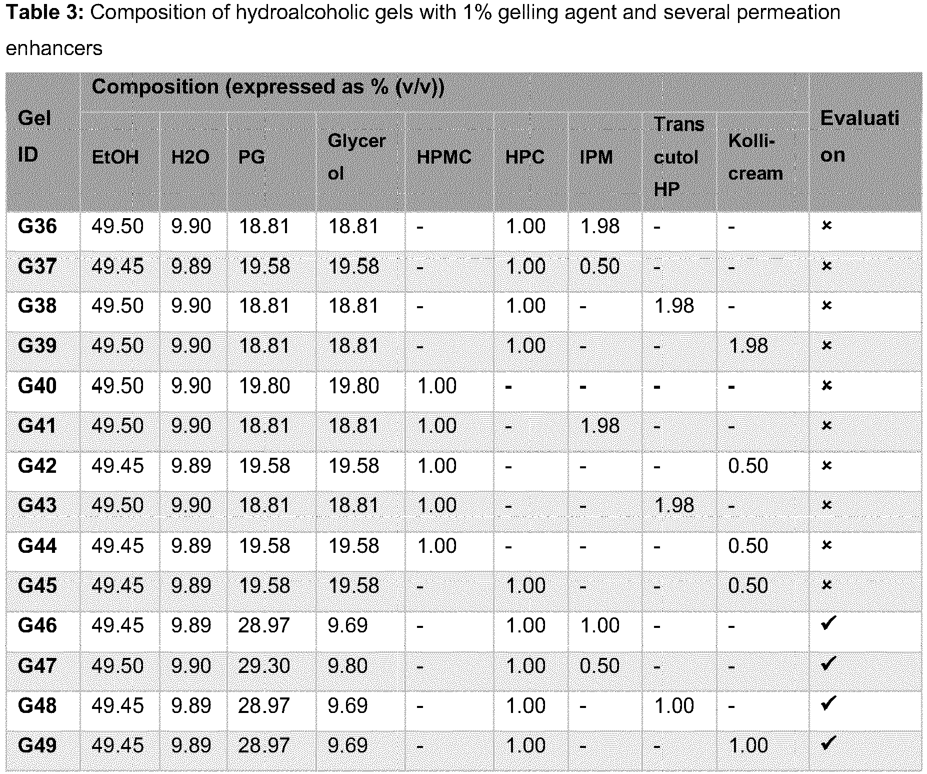

After cosmetic evaluation of these gels, all gels were fine, G46-G49 being slightly better. It was observed that HPMC formed slightly more sticky gels than HPC and that the best PG:glycerol ratio was (3:1) after application of the gel on the skin.

Zein and Gantrez^{™} ES-425 (GES) were then added to the selected gels (G46 to G49) in order to evaluate their compatibility. The resulting combinations can be seen on the following table:

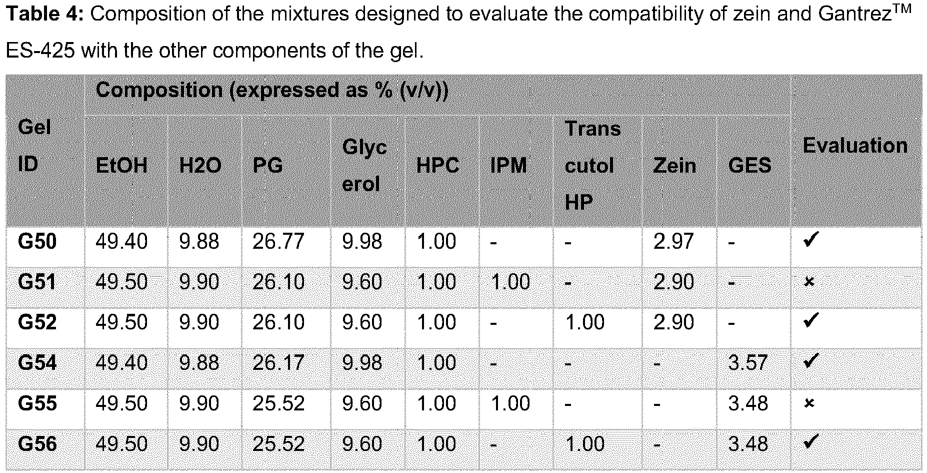

These gels were stored in a climatic chamber at 25°C/60%RH and their macroscopical appearance was re-evaluated after 1 week. The compositions containing IPM (G51 and G55) tended to lose their consistency and had to be discarded. However, gels G50 and G54 (without permeation enhancers) and gels G52 and G56 (with transcutol HP) remained unaltered and displayed good macroscopical properties.

After careful selection of the most appropriate vehicles, several organic phases were prepared by solubilizing TTB in PG, glycerol and DMSO and combining them with various permeation enhancers, surfactants and plasticizers that displayed good solubility of the active. See the preparations in table 5 on the next page:

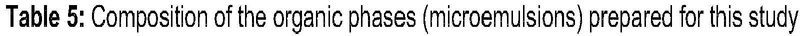

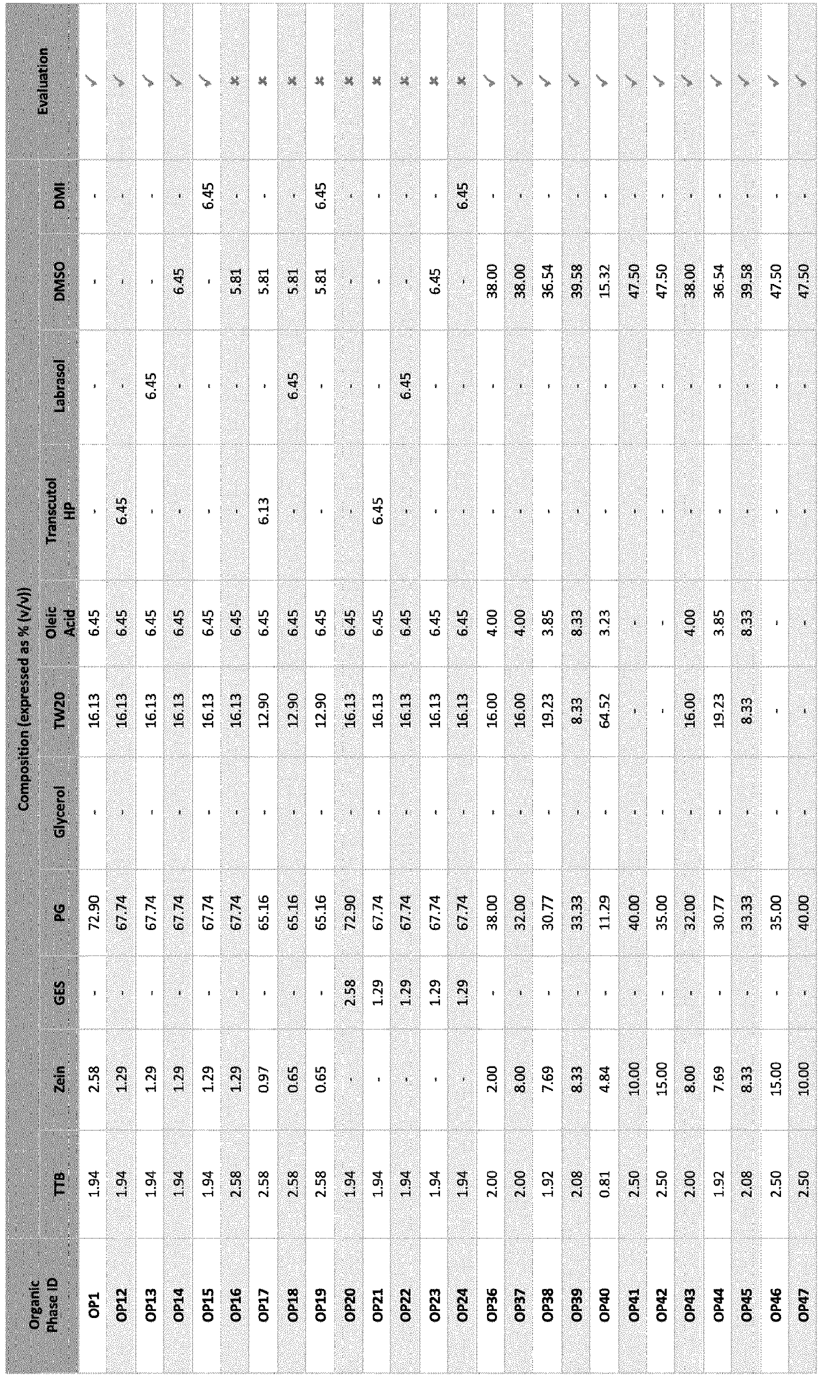

Organic phases OP20 to OP24 had to be discarded as TTB precipitates when in contact with Gantrez^{™} ES-425. However, all the organic phases containing zein were clear and did not show any phase separation, crystallization or aggregation.

Finally, the definitive formulations were prepared combining the previously described organic phases with gel G50 without zein (i.e. the composition displayed in table 4 above including ethanol, water, propylene glycol, glycerol, and HPC). Several permeation enhancers were incorporated, alone and in combination, in order to compare their effect on TTB permeation through the skin in the *ex vivo* percutaneous absorption testing. The objective was to achieve maximum active concentration in the skin while maintaining minimum concentrations in the receptor chamber. Table 6 displays the composition of these formulations.

The polymer concentration of the water-insoluble polymer (zein) in the final formulation was established under 1% (w/w) to form a controlled delivery system in which the active could be released after a relatively short time after product application. The permeation enhancer concentration was also kept under 20% (w/w) to limit the active's systemic absorption.

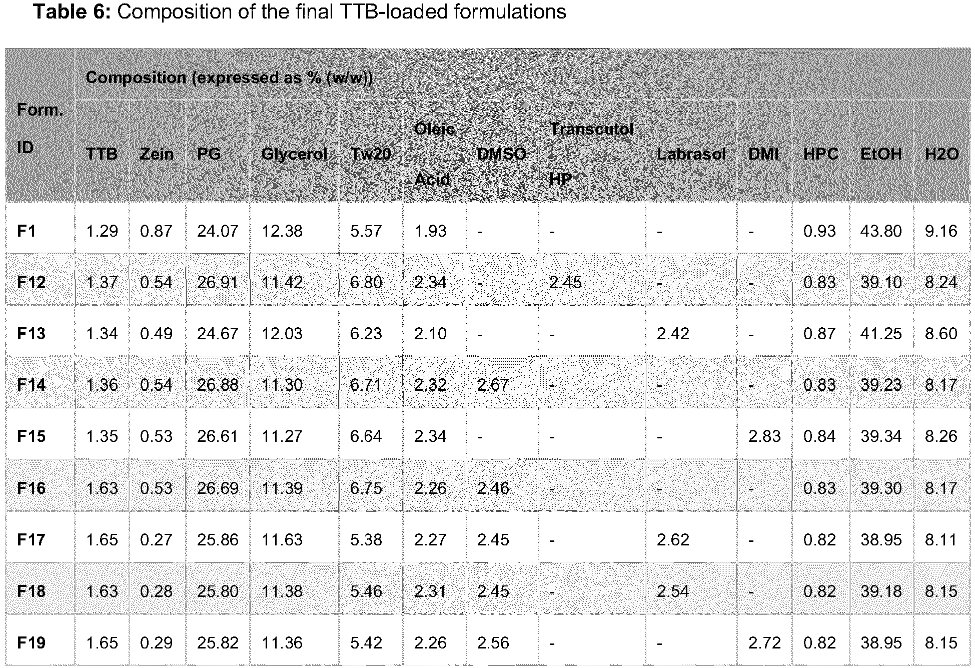

However, in parallel, several formulations were designed with increased concentrations of DMSO (as this was the permeation enhancer that was able to solubilize higher amounts of tiotropium) and also with higher polymer concentrations of the water-insoluble polymer in order to find out whether this combination provides more adequate permeation profiles. See their compositions in table 7 below.

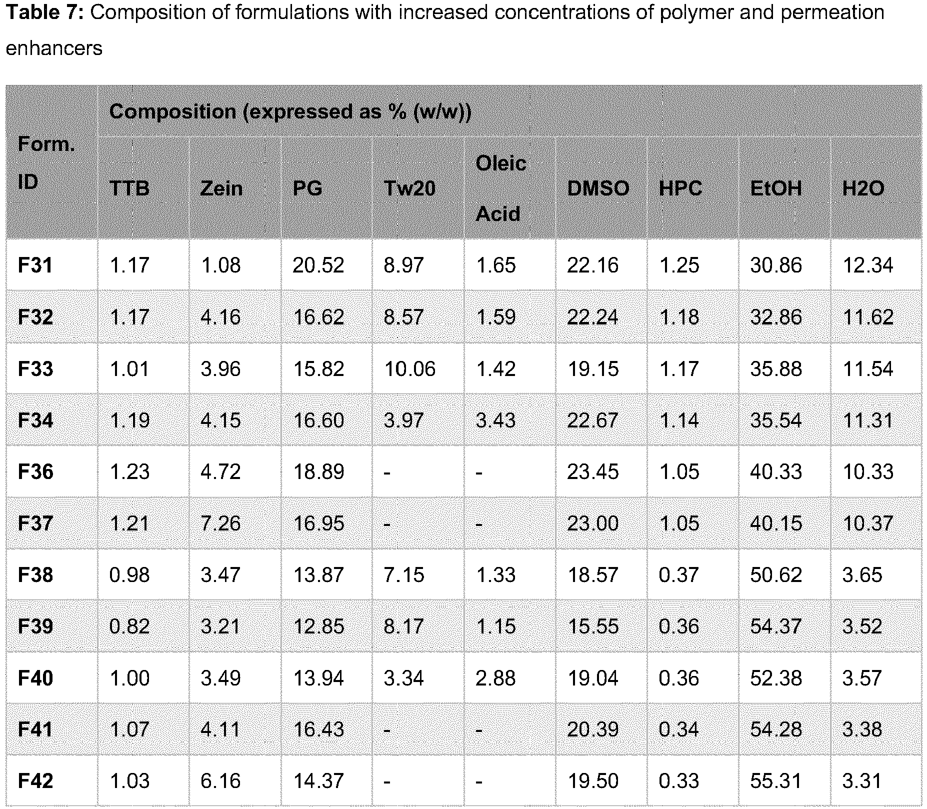

### Strategy 2: Anhydrous gel

In parallel to the first strategy, another formulation approach was explored consisting on the design of anhydrous gel formulations using a gel-forming agent especially designed for water-free products. The composition of these formulations is displayed in table 8 below.

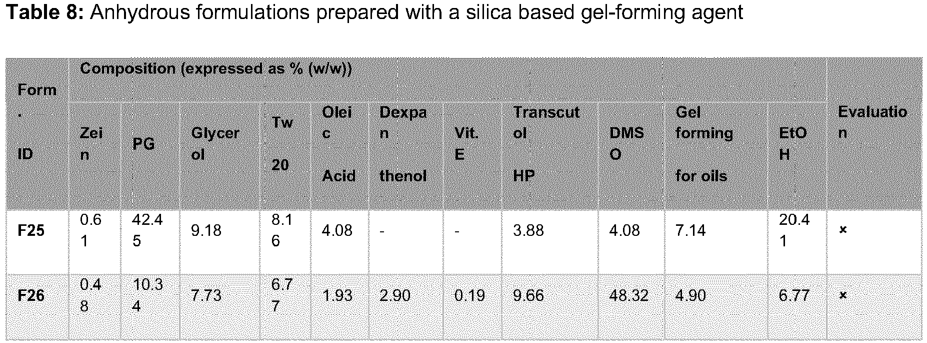

Formulation F25 had high concentrations of propylene glycol and ethanol whilst formulation F26 contained high concentrations of DMSO instead - as a way to compare their appearance. However, both products presented very poor cosmetic properties and displayed an oily and sticky feeling when applied on moist hands.

Three additional anhydrous formulations were prepared by addition of 1% HPC to three previously prepared organic phases.

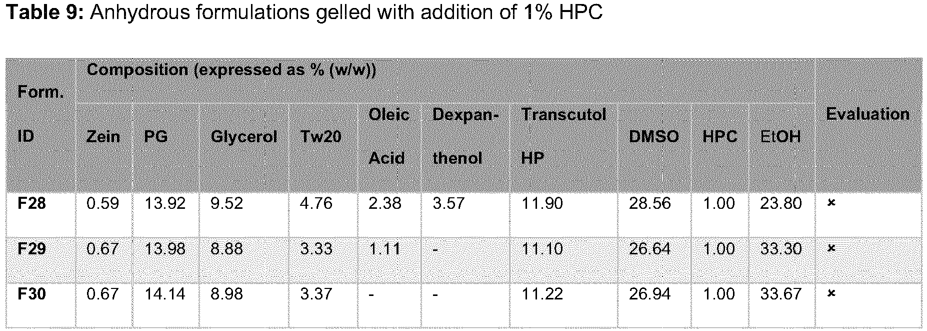

The cosmetic appearance of these formulations was not acceptable either.

This second strategy was therefore discontinued.

### 1. Optimization and physicochemical characterization

### 1.1 Analytical method development and implementation

Once the most appropriate chromatographic conditions were established for the identification of tiotropium in the formulations, the basic analytical tests were conducted in order to validate the analytical method for linearity, accuracy and precision in the concentration range between 1.00 and 50.00 µg/mL.

The calibration curve obtained for the analytical method was: Y= 191.37*X - 5.7017 with a correlation coefficient (R²) of 0.9999.

This analytical method was used to accurately determine the concentration of active in each of the designed prototypes and monitor its evolution with time under the pre-established stability conditions.

For TTB quantification, approximately 25 mg of each formulation were accurately weighed in an analytical balance into a glass vial. The active was extracted from the formulations by vigorous stirring with a mixture of phosphate buffer and methanol in a ratio 70:30. Once the formulation was completely dispersed, the mixture was transferred to a volumetric flask and filled to the mark with the same solution. A small fraction was filtered through 0.22 µm membranes and then injected onto the HPLC column.

### 1.2 Macroscopical evaluation

All the formulations were evaluated for macroscopical appearance. The following specifications were required: clarity, absence of aggregation, active crystallization and/or phase separation.

Increasing zein concentrations result in increasing yellow coloration. All the formulations were clear and did not present any macroscopical crystals. No aggregates or phase separation were observed, after preparation or after release from any of the evaluated stability conditions.

However, when zein was removed from the formulations the formation of macroscopical crystals was observed. Thus, without being bound by a particular theory, it appears that zein serves to avoid precipitation, at least at higher TTB concentrations.

### 1.3 Particle Size measurement

The formulations were diluted with water and their particle size measured on a Brookhaven 90S Dynamic Light Scattering equipment. Only formulations with particle size lower than 350 nm and polydispersity index lower than 0.35 were considered acceptable.

All the samples were analysed at the time of their preparation and also after 72h as a preliminary approach to their stability.

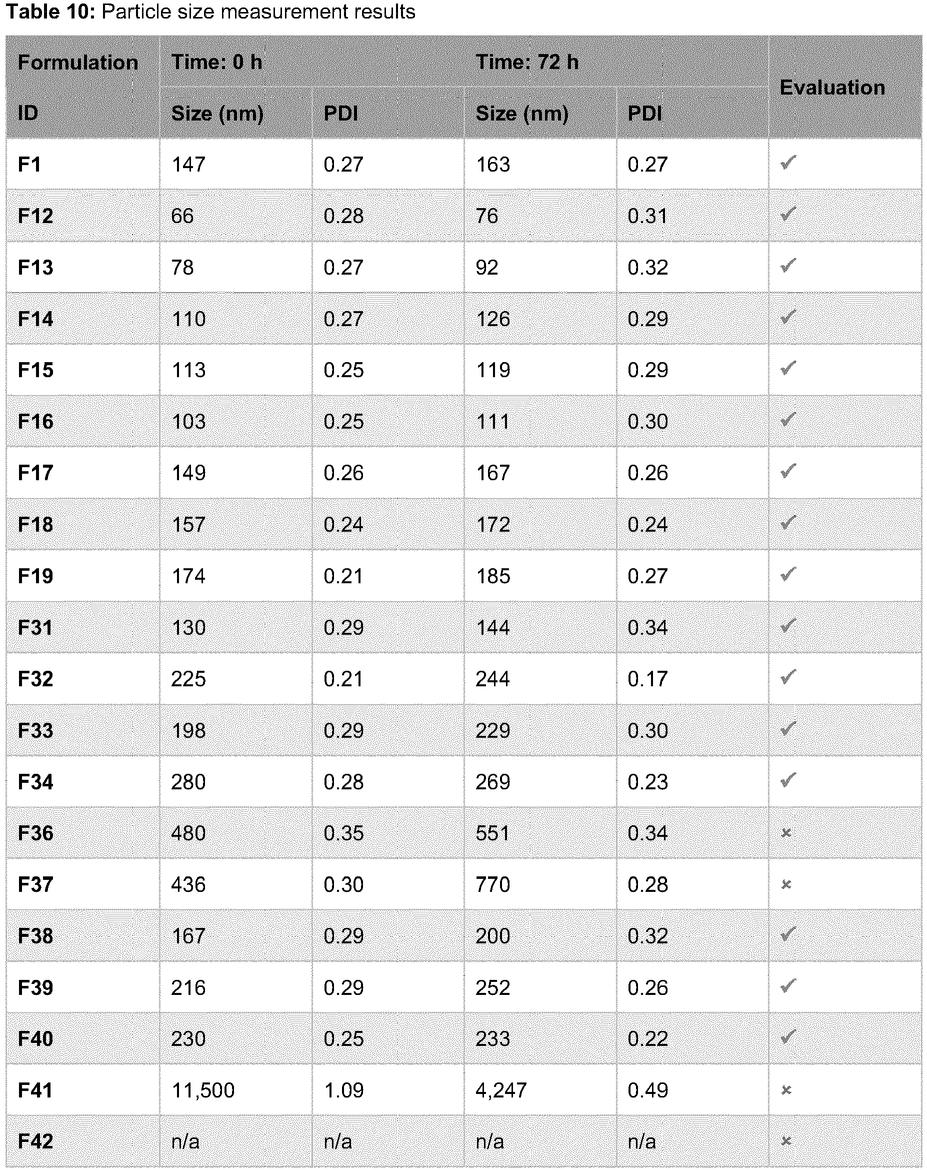

All formulations were acceptable. Formulations F36, F37, F41 and F42 do not contain any surfactants and therefore formed larger particles and aggregates upon contact with water. Theses prototypes will be discarded on the final selection.

All the formulations presented an opalescent effect upon dilution with water which was indicative of the presence of particles. In addition, formulations with higher zein concentration presented a whitish chalky appearance due to the precipitation of the polymer upon contact with water.

### 1.4 Stability

To check the formulations' behaviour and discard those that could lose their properties, small aliquots of each of them were placed into amber glass vials, sealed with PTFE lids and stored under several conditions.

Firstly, an initial TTB quantification by means of HPLC-UV was carried out. The following table represents the initial quantification results. A comparison was made between the theoretical concentration and the experimental one obtained after HPLC analysis. A ± 10 % tolerance interval was considered acceptable.

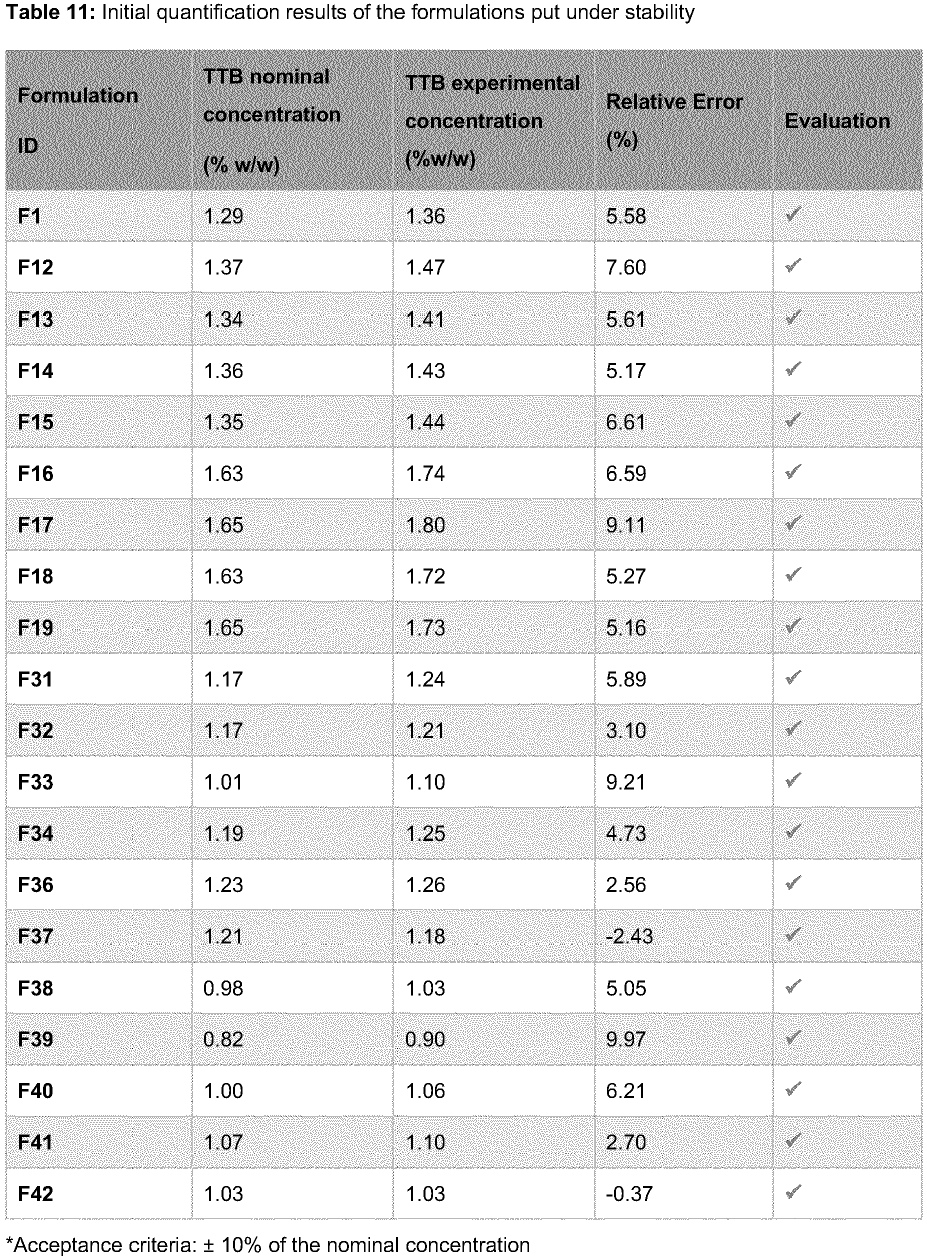

### 1.4.1 Screening test

The first stability test consisted of a preliminary evaluation after 5 days of storage at 4 °C. The vials were taken out of the fridge and left to reach room temperature for a few hours. They were then analysed as previously described. Table 12 below shows the quantification results.

No changes in appearance were observed in any of the formulations. There was no phase separation, no aggregation and no changes in colour or turbidity.

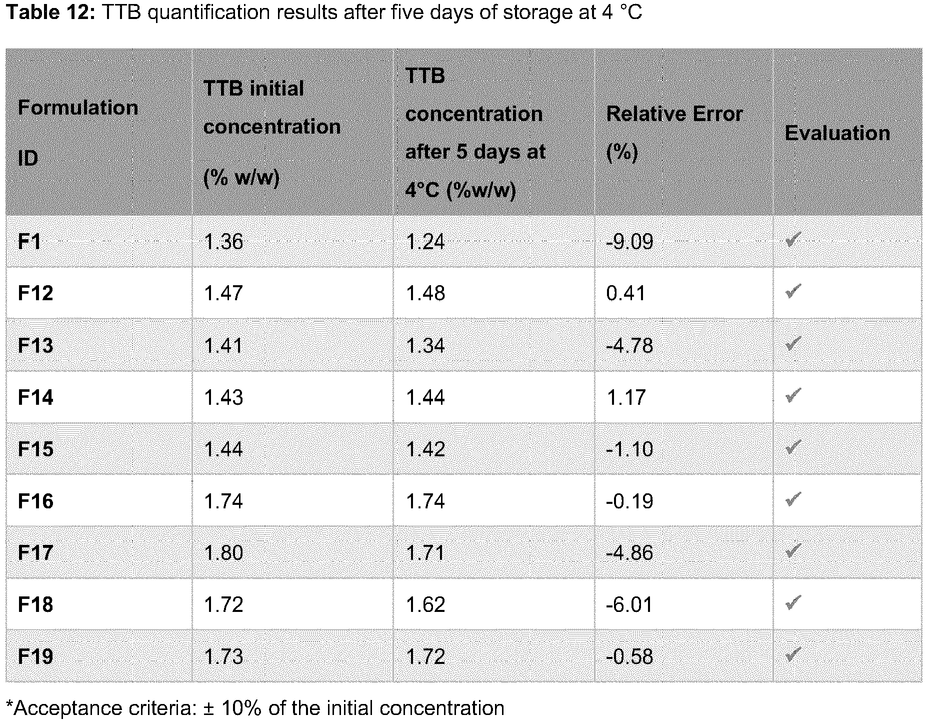

Therefore, all the formulations complied with the previously established acceptance criteria after five days of storage at 4°C.

### 1.4.2 Stress conditions

Another aliquot of the prototypes was subjected to four stress cycles, each of them consisting of 2h heating at 75 °C in an autoclave followed by overnight storage at -20 °C. After the last cycle, the formulations were analysed for macroscopical appearance as well as TTB content.

No changes in appearance were observed in any of the formulations. There was no phase separation, no aggregation and no changes in colour or turbidity. Table 13 below shows the quantification results.

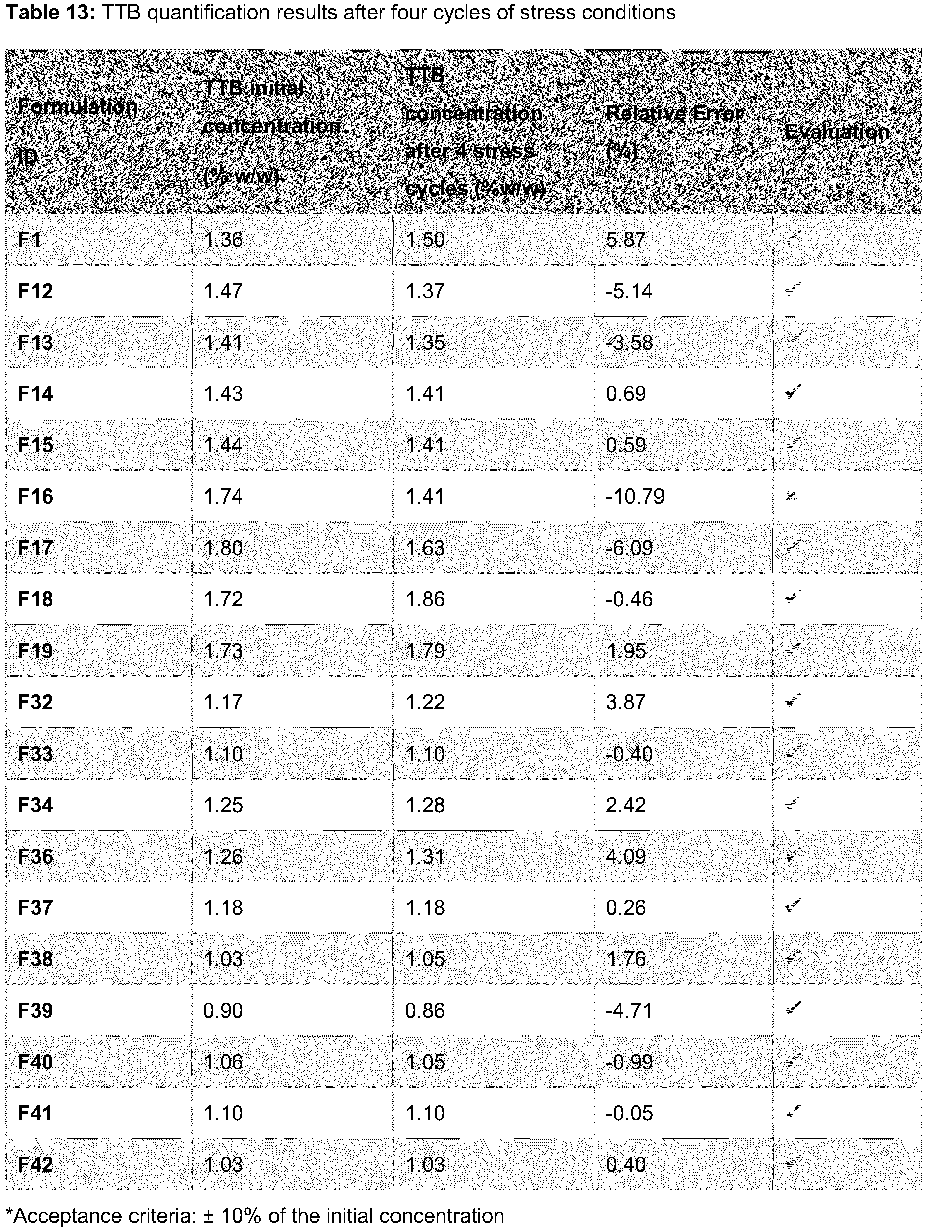

As it can be observed only formulation F16 showed a difference between the initial and final concentration greater than 10%.

### 1.4.3 Stability at 4 °C

The aliquots stored at 4°C were periodically analysed to monitor their evolution over time. Table 14 below shows the quantification results after four months of storage.

The formulations were also analysed for macroscopical appearance. No changes in appearance were observed in any of the products. There was no phase separation, no aggregation and no changes in colour or turbidity.

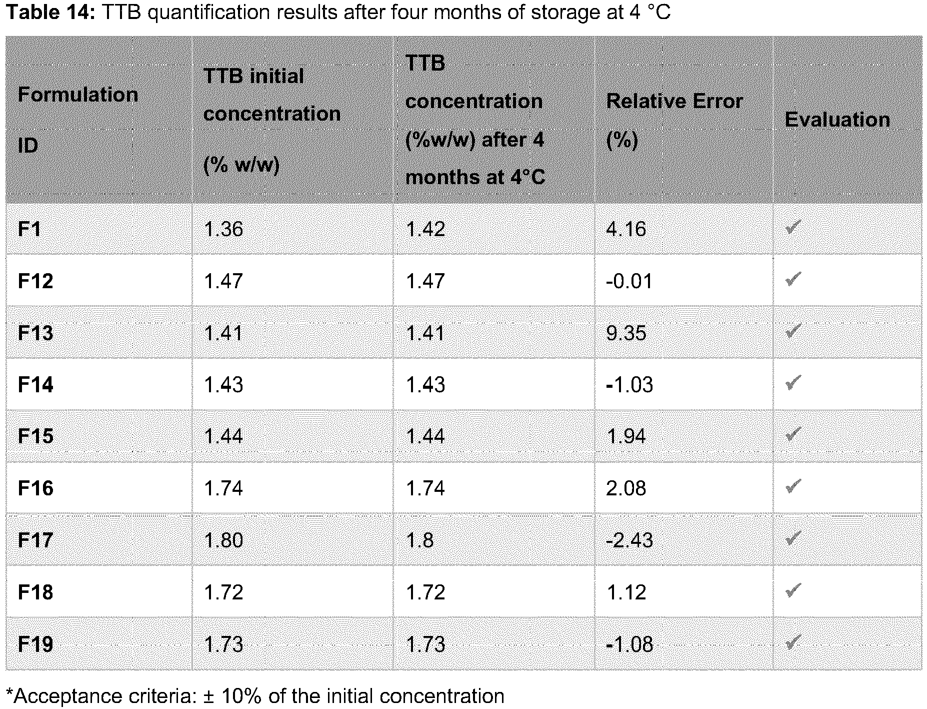

Therefore, all the formulations complied with the previously established acceptance criteria after four months of storage at 4°C.

### 1.4.4 Stability at 25 °C/60% RH

The aliquots stored in the climatic chamber at 25°C and 60% RH were periodically analysed in order to monitor their evolution over time. Table 15 below shows the results after four months of storage.

The formulations were also analysed for macroscopical appearance. No changes in appearance were observed in any of the products. There was no phase separation, no aggregation and no changes in colour or turbidity.

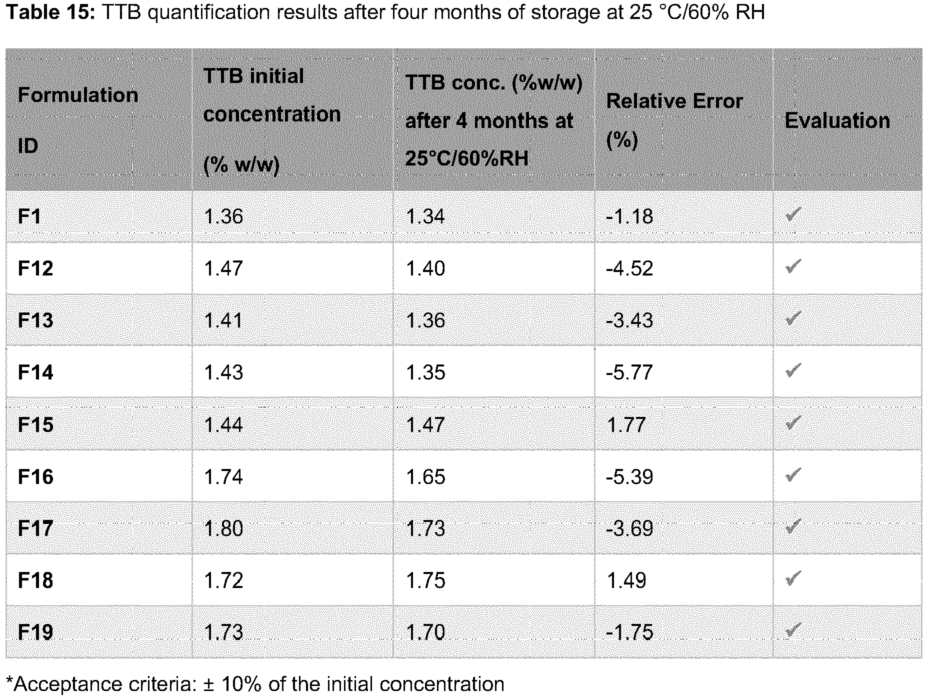

Therefore, all the formulations complied with the previously established acceptance criteria after four months of storage at 25°C and 60% RH.

### 1.4.5 Stability at 40 °C/75% RH

The aliquots stored in the climatic chamber at 40°C and 75% RH were periodically analysed in order to monitor their evolution over time. Table 16 below shows the results after three months of storage.

The formulations were also analysed for macroscopical appearance. No changes in appearance were observed in any of the products. There was no phase separation, no aggregation and no changes in colour or turbidity.

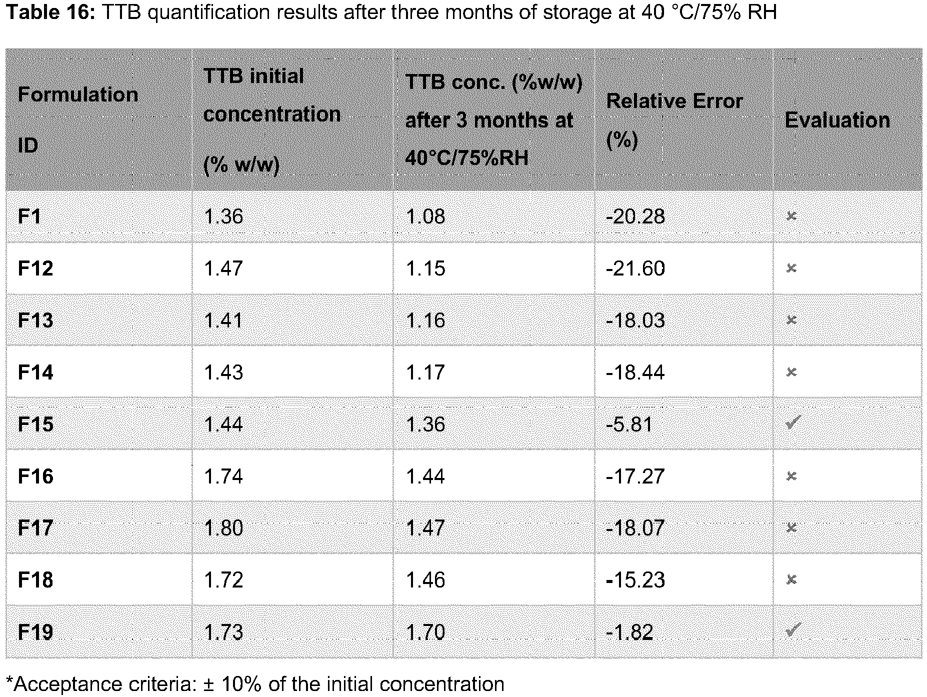

All formulations are acceptable, but F15 and F19 had a remarkable and surprising stability. Therefore, only formulations 15 and 19 complied with the previously established acceptance criteria after three months of storage at 40°C and 75% RH and were analysed again after four months.

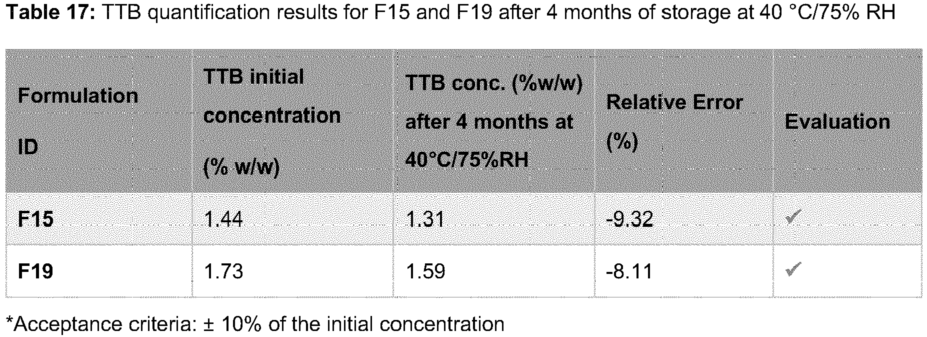

These formulations unexpectedly comply with the acceptance criteria after four months of storage at 40°C and 75% RH.

### 1.5 Ex vivo percutaneous absorption study

As indicated before, several preliminary *ex vivo* studies have been performed in order to compare the percutaneous absorption of the selected formulations. Tiotropium exerts its action in the sweat glands located in the dermal layer of the skin, but it is also desirable to minimize its systemic escape (i.e. its concentration in the receptor chamber).

Formulations F1 and F12 to F19 contained different permeation enhancers and therefore it was necessary to determine whether synergic effects could be found and/or whether certain permeation enhancers were more effective than others for this active. Formulation F31 contained a higher concentration of DMSO and F37 a higher concentration of polymer. In addition, an internal control was included in the study to guarantee the integrity of the experiment.

To find the most appropriate endpoint for the percutaneous absorption study, a Franz diffusion cell study was performed in which the permeation profile of a solution of TTB in DMSO (internal control) and a formulation without permeation enhancers were compared. Samples of the receptor chamber were collected and analysed at different timepoints (0, 0.5, 1, 2, 4, 6, 8, 12 and 22 hours) while the skin and wash fluids were analysed at the end of the experiment. Three replicates per formulation were assayed.

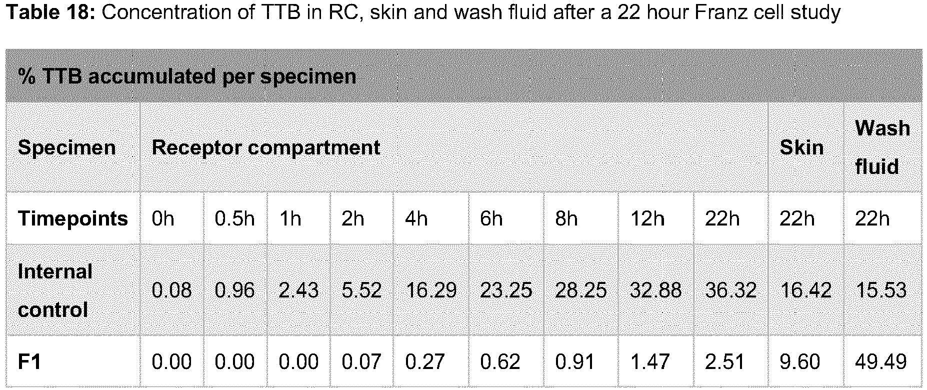

After evaluation of these results, it was established that if differences between formulations existed, six hours was time enough to quantify them on the HPLC and, at the same time, it was an endpoint that allowed us to manage the processing of the samples in one working day.

Subsequently, several mini-Franz cell percutaneous absorption studies were carried out with a six hour endpoint and using six replicates per formulation. The diffusion area of these cells was 1.76 cm² and therefore approximately 18 µL of product needed to be applied (in each case the corresponding weight was accurately measured using an analytical balance). Each piece of skin was massaged for 15 seconds with a plastic rod after product application to mimic real-life conditions when the product is applied on a patient's hands. The amount of TTB adhered to the rod was also quantified by HPLC.

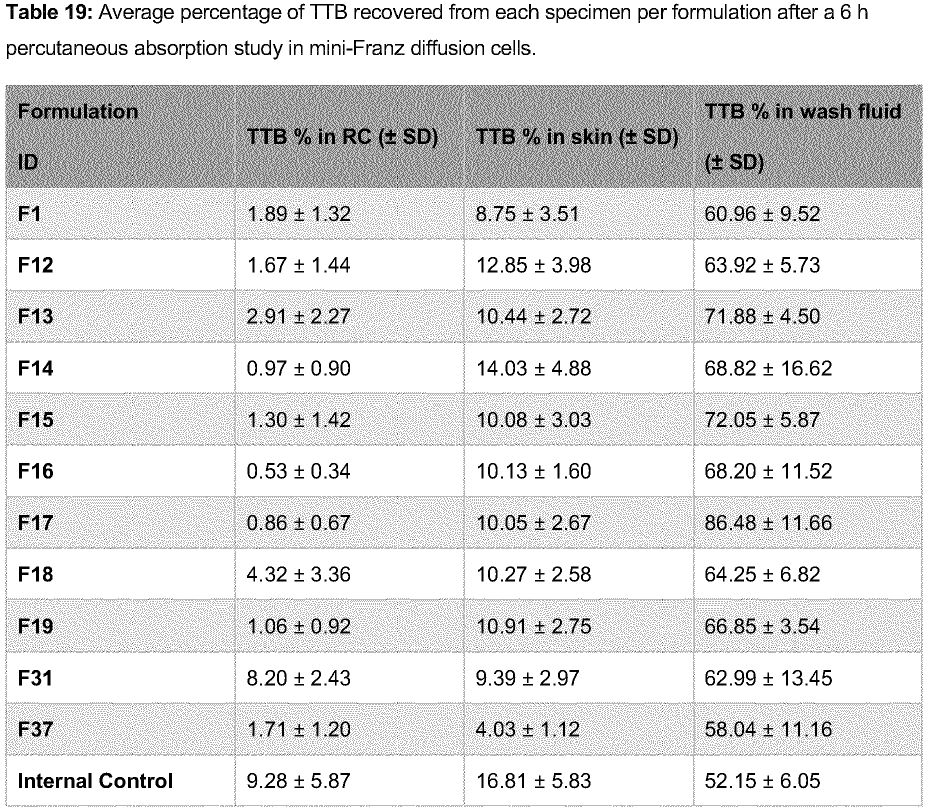

Based on these results, it can be concluded that increasing the permeation enhancer up to approximately 20 % (w/w) (formulation F31) does not result in an increment of TTB concentration on skin but it does on receptor chamber after six hours of exposure. On the other hand, as hypothesized, increasing the polymer concentration of the water-insoluble polymer (formulation F37) does not vary the active's concentration in either receptor chamber or skin after six hours of exposure.

A 24 hour long *ex vivo* experiment was then performed with formulations F31 and F37 in order to determine the effect of increasing the permeation enhancer and polymer upon longer exposure periods. The results can be found in table 20 below.

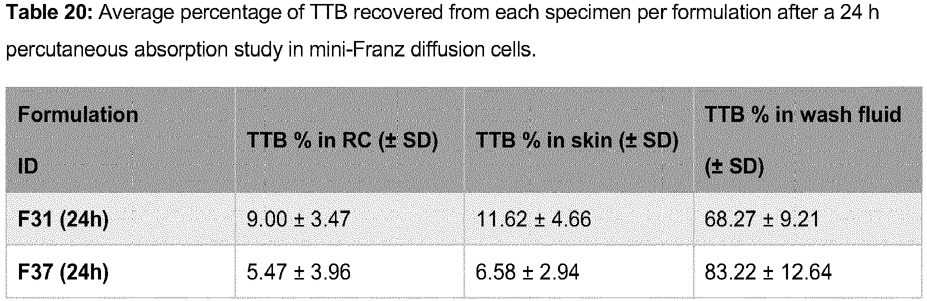

Increasing the concentration of DMSO and/or zein does not result in an increase of TTB in skin after either 6 or 24 hours as expected.

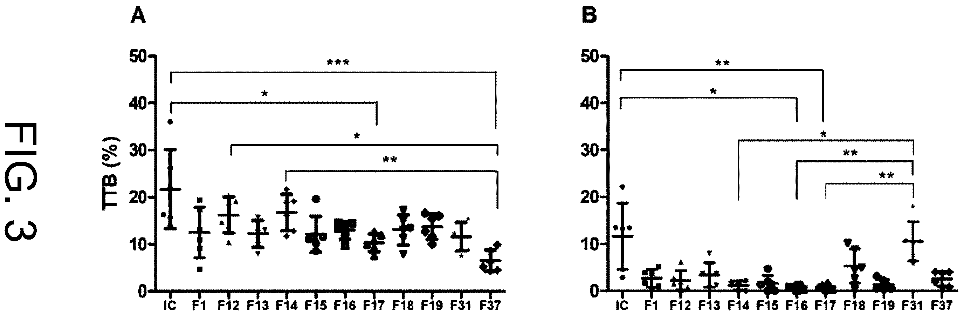

A statistical analysis was performed on these data to find out whether significant differences could be found between formulations. For that purpose, the percentages of TTB in skin and receptor chamber were normalized and are represented in Figure 3.

The differences between the prototypes were evaluated using a Kruskal-Wallis test with Dunn's multiple comparison *post-test* and considered significant when p < 0.05. The statistical analysis was performed using GraphPad Software.

The objective was to achieve maximum active concentration in the skin while maintaining minimum concentrations in the receptor chamber.

Looking at the receptor chamber fluid, formulations F16 and F17 displayed significantly lower concentrations of TTB than the internal control (a hydroalcoholic gel with TTB in DMSO) and formulations F14, F16 and F17 significantly lower concentrations of TTB than F31 (an oil-in-water emulsion gel with high DMSO content).

When it comes to skin, only formulation F17 showed significantly lower concentration of TTB than the internal control whereas F12 and F14 displayed significantly higher TTB concentration than F37 (an oil-in-water emulsion gel with high polymer content). This observation was remarkable and provided surprising TTB concentration to the skin.

### Comparison with and without zein

Formulations F12 and F15 from Table 6 were tested at lower concentrations of TTB. The following formulations (F12 and F15 with and without zein) were tested:

**Table 21**

| Form ID | **Composition (expressed as % (w/w))** | | | | | | | | | | |
|---|---|---|---|---|---|---|---|---|---|---|---|
| | **TTB** | **Zein** | **PG** | **Glycerol** | **Tween 20** | **Oleic Acid** | **Transcutol HP** | **DMI** | **HPC** | **EtOH** | **H2O** |
| F12 | 0.10 | 0.54 | 26.36 | 11.25 | 6.94 | 2.57 | 2.58 | - | 0.85 | 40.44 | 8.37 |
| F12 w/o zein | 0.10 | - | 26.96 | 11.26 | 6.61 | 2.25 | 2.76 | - | 0.85 | 40.80 | 8.40 |
| F15 | 0.52 | 0.53 | 25.61 | 11.52 | 6.37 | 2.30 | - | 4.18 | 0.81 | 40.20 | 7.98 |
| F15 w/o zein | 0.50 | - | 25.93 | 11.24 | 7.35 | 2.48 | - | 3.99 | 0.80 | 39.83 | 7.88 |

These compositions were tested in an *ex vivo* percutaneous absorption study in a Franz cell as described above, except that the porcine skin samples were replaced with skin from a human donor (Caucasian female, 55 years old, BMI 27, location: abdomen). Furthermore, the applied dose was 5 µl/cm² instead of 10 µl/cm².

The distribution of TTB was the following:

**Table 22**

| | **Epidermis** | **Dermis** | **Epidermis + Dermis** | **Receptor chamber** |
|---|---|---|---|---|
| F12 | 12.3% | 3.9% | 16.2% | 0.6% |
| F12 w/o zein | 6% | 3% | 9% | 0.34% |
| F15 | 4% | 1.2% | 5.2% | 0.19% |
| F15 w/o zein | 4.7% | 0.8% | 5.5% | 1.9% |

These results demonstrate that good retention in the epidermis/dermis can be achieved with and without zein in the formulation, the systemic circulation being limited in both cases.

The stability of the four formulations was furthermore tested as described above:

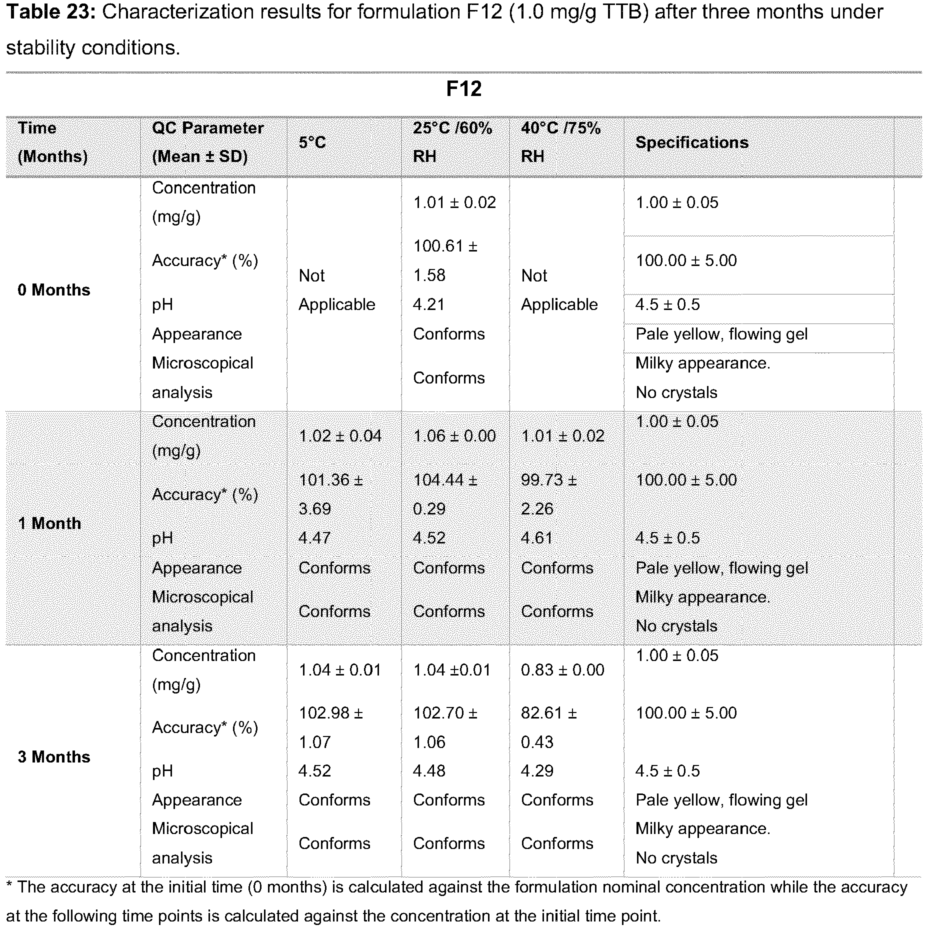

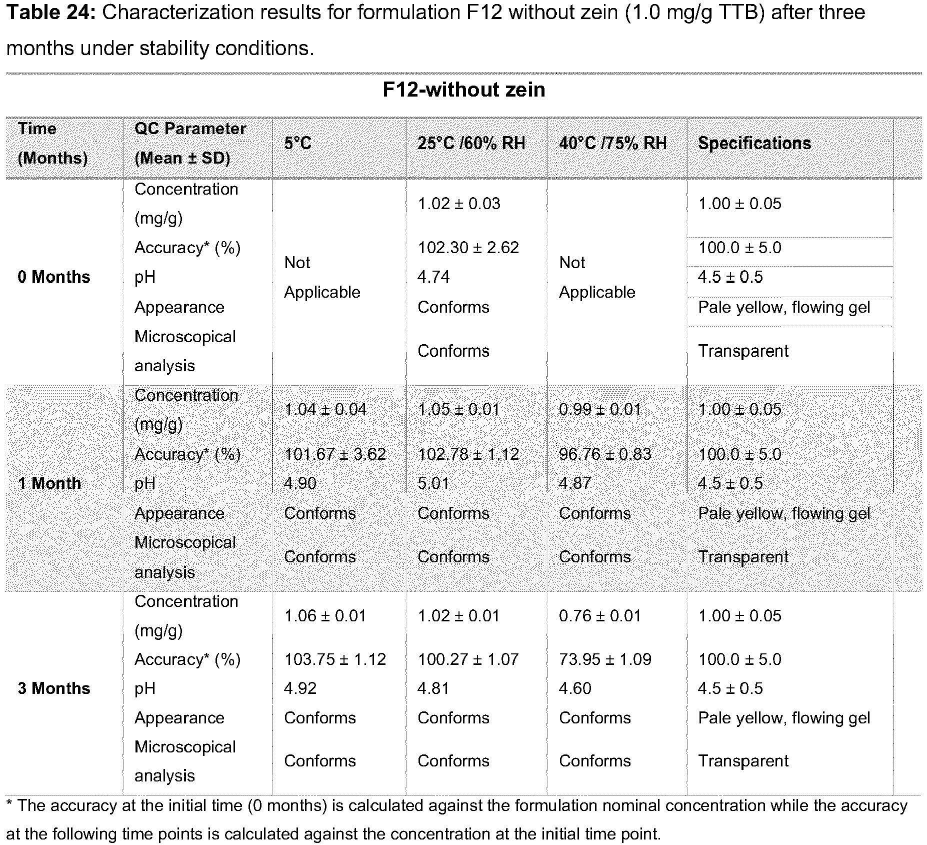

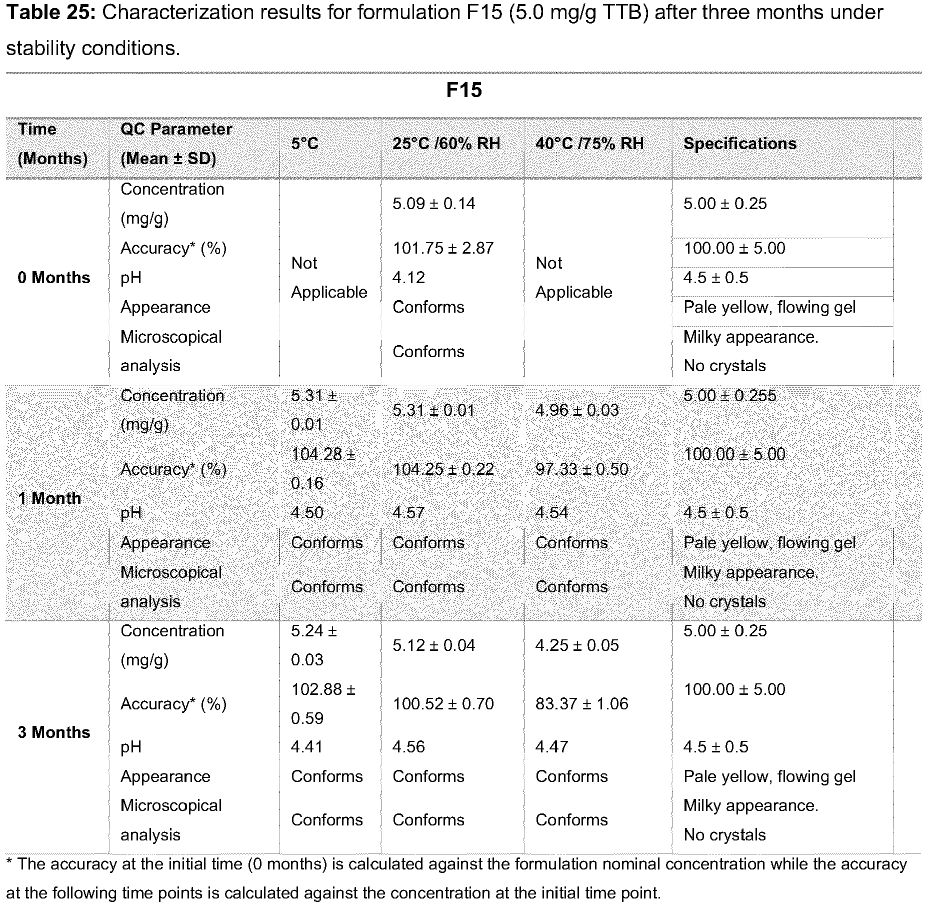

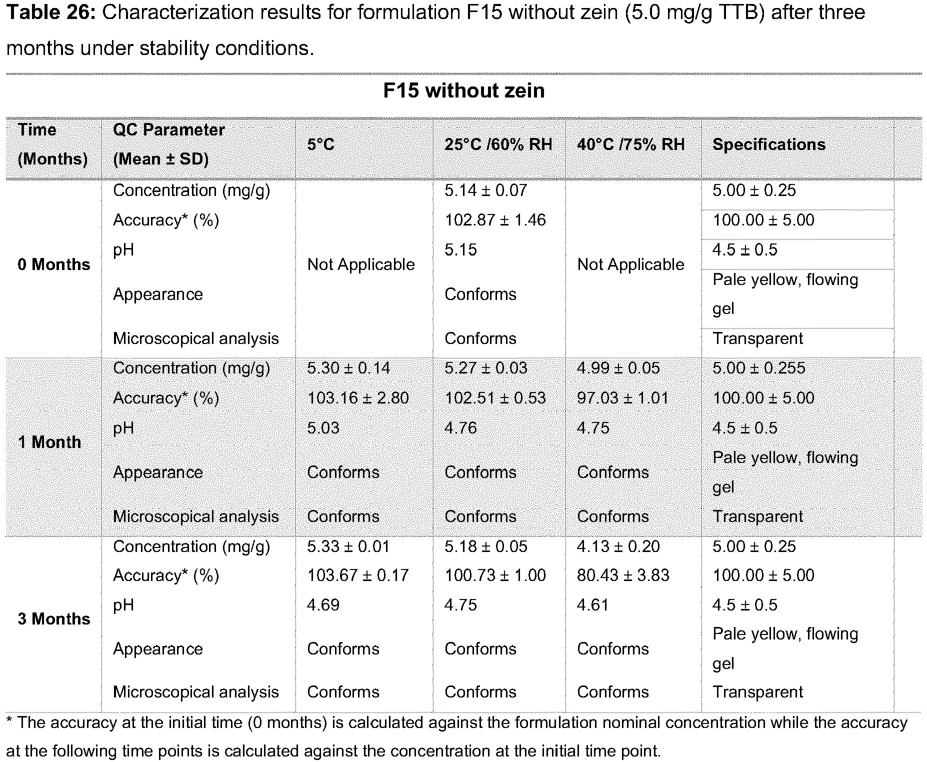

Thus, all the formulations are stable, except under the most accelerated conditions after 3 months.

### Conclusion of the experiments

- In order to achieve a formulation that complied with the requirements, fifty-six gels and forty-seven organic phases were developed that resulted in many promising prototypes.
- Two different formulation strategies were explored: an oil-in-water emulsion gel as well as an anhydrous one. The latter had to be discarded due to its poor cosmetic properties.
- The previously obtained formulations were all zein-based as a chemical incompatibility between Gantrez^{™} ES-425 and TTB was found during the development.
- Regarding the stability of the formulations:
   ∘ All the formulations complied with the stability criteria after 4 months of storage at 4 °C and also at 25 °C / 60% RH.
   ∘ All formulations except F16 withstood four cycles of stress conditions.
   ∘ Only F15 and F19 remained stable after four months of storage at 40 °C and 75% RH.
- Several *ex-vivo* percutaneous absorption studies were carried out that confirmed that the most adequate polymer concentration of the water-insoluble polymer was under 1% (w/w) to form a controlled delivery system in which the active could be released after a relatively short time after product application and that the permeation enhancer (PE) concentration is preferably under 20 % (w/w) to limit the active's systemic absorption.
- Regarding the receptor chamber fluid, formulations F16 and F17 displayed significantly lower concentrations of TTB than the internal control (a hydroalcoholic gel with TTB in DMSO) and formulations F14, F16 and F17 significantly lower concentrations of TTB than F31 (an oil-in-water emulsion gel with high DMSO content).
- When it comes to skin, only formulation F17 showed significantly lower concentration of TTB than the internal control whereas F12 and F14 displayed significantly higher TTB concentration than F37 (an oil-in-water emulsion gel with high polymer content).

## Claims

1. A dermatologically acceptable oil-in-water emulsion gel comprising tiotropium bromide.

2. A gel according to claim 1 comprising a water-soluble polymer selected from cellulose derivatives, such as hydroxyethyl cellulose, hydroxypropyl cellulose or hydroxypropyl methylcellulose.

3. A gel according to any one of the preceding claims comprising one or more permeation enhancers.

4. A gel according to claim 3, wherein the concentration of the one or more permeation enhancers is in the range 0.1 to 20% (w/w), such as in the range 0.2 to 10% (w/w), e.g. in the range 0.3 to 5.0% (w/w), such as in the range 0.5 to 3.0% (w/w).

5. A gel according to claim 1 or 2, wherein tiotropium bromide is encapsulated by or adhered to a water-insoluble polymer.

6. A gel according to claim 5, wherein the water-insoluble polymer is zein.

7. A gel according to any one of the preceding claims comprising one or more non-ionic surfactants, such as polysorbates, polyoxyglycerides, fatty acid esters or polyethoxylated castor oil.

8. A gel according to any one of the preceding claims, wherein the concentration of tiotropium bromide in the gel is from about 0.01% to about 5.0% w/w such as from about 0.025% to about 3.5% w/w, from about 0.05% to about 2.5% w/w, from about 0.1% to about 2.5% w/w, from about 0.5% to about 2.5% w/w such as from about 0.75% to about 2.0% w/w, based on the total weight of the gel.

9. A gel according to any one of the preceding claims comprising one or more solvents selected from propylene glycol, glycerol, and ethanol.

10. A gel according to any one of the preceding claims comprising ethanol in a concentration of from 25% to 55% w/w based on the total weight of the gel.

11. A gel according to any one of the preceding claims comprising water in a concentration of from 5% to 15% w/w, ethanol in a concentration of from 25% to 55% w/w, glycerol in a concentration of from 7.5% to 15% w/w, and propylene glycol in a concentration of from 15% to 30% w/w based on the total weight of the gel.

12. A gel according to any one of the preceding claims for use in the treatment or prevention of hyperhidrosis.

13. A gel according to any one of claims 1-11 for use as an anti-perspirant.

14. A method for preparing an oil-in-water emulsion gel comprising tiotropium bromide, the method comprising
i) preparing an hydroalcoholic gel comprising one or more water-soluble cellulose derivatives,
ii) preparing a organic phase comprising tiotropium bromide,
iii) mixing the hydroalcoholic gel with the microemulsion to obtain said oil-in-water emulsion gel.

15. A method according to claim 14, wherein the hydroalcoholic gel is prepared by dissolving the water-soluble cellulose polymer in water, then adding ethanol and optionally other topically acceptable agents.
